# EUROPEAN PATENT APPLICATION

(11) **EP 4 826 663 A1**
(43) Date of publication of application: **21.10.2026**
(21) Application number: 26171479.4
(22) Date of filing: 09.04.2026
(51) Int. Cl.: G06T 12/30

(54) **DEEP LEARNING MODEL TRAINING FOR CORRECTION OF LATERAL CBCT TRUNCATION WITH DATA-CONSISTENCY ENFORCEMENT**

(30) Priority: 14.04.2025 US 202519178435
(71) Applicant: Siemens Healthineers International AG, 6312 Steinhausen (CH)
(72) Inventor: PETERLIK, Igor, 5405 Dättwil (CH); SHEN, Sangyu, Palo Alto, 94304 (US); STRZELECKI, Adam, 5405 Dättwil (CH); SEGHERS, Dieter Marc Denis, 5405 Dättwil (CH)
(74) Representative: Mathisen & Macara LLP

(57) **Abstract**

A method for training a machine learning model to de-truncate volumes. The method receives a non-truncated volume of an anatomical region, the non-truncated volume representing imaging data within a full scan field of view (SFoV). A non-truncated stack of projections 1204 is simulated based on the non-truncated volume. The non-truncated stack of projections is truncated by changing at least one visual attribute of the non-truncated stack of projections outside a predefined SFoV threshold, thereby generating a set of simulated truncated volumes 1208. Training pairs each comprising the non-truncated volume 1206 paired with a truncated volume 1210 are formed, wherein the non-truncated volume is designated as ground truth. The machine learning model is trained 1258 using the set of training pairs, wherein the machine learning model is configured to infer missing regions in truncated volumes.

## Description

### TECHNICAL FIELD

This application pertains generally to the field of medical imaging and, more specifically, to systems and methods for correction of truncated volumes, such as correction of lateral CBCT truncation.

### BACKGROUND

Computed Tomography (CT) scans are critical for providing detailed, non-invasive imaging of internal structures of patients, enabling accurate diagnosis, treatment planning, and evaluation in medical applications. The process begins with emitting X-ray beams from a rotating source that pass through the patient and are detected by a detector positioned on the opposite side. The received X-ray beams may have variations in attenuation caused by differences in tissue density, and these variations may be used to reconstruct a detailed visualization of the internal structure of a patient. However, the X-ray beams may not capture all of the necessary angles of a patient if the patient is too large for a CT scanner or is incorrectly positioned. In this case, the beam may only pass through part of the patient at some angles. As a result, the captured data may be insufficient to construct a complete volume of the patient.

Conventional methods of addressing incomplete CT scans involve many challenges. Hardware-based approaches, such as using larger imagers or lateral shifts, are constrained by physical and logistical challenges, including increased system complexity and longer scan times. Traditional algorithmic methods, such as sinogram correction and ellipse-fitting, fail to maintain 3D consistency in images and often produce suboptimal results. These conventional algorithmic methods merely provide estimates of the portion of the patient that was only captured at some angles. As a result, the estimates using these conventional methods can lack accuracy, particularly as distance from the portion of the patient that was fully captured increases, the data captured in the scan becomes more incomplete. Furthermore, traditional algorithmic methods may make assumptions, such as assuming internal structures of a patient, follow an elliptical form or assuming the mass of a scanned object is constant under all angles, which can lead to inaccuracies in generated volumes. Therefore, conventional methods of addressing incomplete CT scans face technical challenges.

### SUMMARY

For the aforementioned reasons, there is a need for de-truncation methods and systems that leverage truncated scans of a patient to generate complete volumes representing the internal structure of a patient. There is a need for a technical solution for generating complete volumes based on truncated imaging data. What is needed is systems and methods that can reliably train and execute a model to generate complete volumes based on truncated volumes, increasing the accuracy of the volumes, and therefore resulting in improved visualization of the internal structures of a patient. More detailed and accurate representations of these internal structures may lead to improved detection of abnormalities, better treatment planning (e.g., due to more precise identification of tumor size, shape and location), enhanced monitoring, and reduced need for invasive evaluations (e.g., exploratory surgeries).

In some embodiments, the methods and systems discussed herein train and use a neural network to generate data to correct estimations of a volume that are made on incomplete imaging data. By simulating truncated volumes based on existing complete volumes, the system may train a neural network to make corrections to a truncated area.

According to a first aspect of the invention, there is provided a method as defined in claim 1. Optional features are specified in the dependent claims.

According to a second aspect of the invention, there is provided a computer-readable storage medium as defined in claim 9. Optional features are specified in the dependent claims. A computer system may comprise the computer-readable storage medium.

According to a third aspect of the invention, there is provided a computer system as defined in claim 14. Optional features are specified in the dependent claim.

The systems and methods may generate simulated truncated volumes, as pairs of truncated volumes may be difficult to acquire. For example, truncated volumes of a patient that is too large for a CT scanner to capture a non-truncated scan may exist, however as a result a corresponding non-truncated volume may not exist due to the patient being too large for the CT scanner. Furthermore, truncated volumes of a patient that is scanned in an incorrect position may exist, however an additional scan may not be performed due to the additional radiation dose it would require. Neural networks require large amounts of high-quality imaging data, and as a result systems and methods for simulating training data sets including pairs of truncated and full volumes are necessary due to the lack of existing training data.

A stack of projections may be generated based on a volume. This stack of projections may simulate the stack of projections that a CT scanning system would generate based on scanning a patient. However, in some examples, the stack of projections may not consider physical phenomena. As a result, the training volumes may include less features for the neural network to analyze, resulting in more efficient training.

A truncated stack of projections may be generated to emulate truncation that would result from a patient being partially out of view of the beam at some angles. A full and truncated volume may then be generated based on the stack of projections. The neural network may be trained on a dataset comprising a plurality of full and truncated volumes. The volumes may contain less data than their corresponding stacks of projections, therefore by training the neural network on volumes, as opposed to stacks of projections, the neural network may be more efficiently trained.

The trained neural network may be incorporated into a U-NET model. For example, the U-Net model may incorporate the trained neural network as a generator and a discriminator model that determines which parts of a volume are real and which parts are generated. By using this adversarial training method, the generator may produce more realistic textures in an effort to manipulate the discriminator into making incorrect predictions. As a result, de-truncated volumes may include more realistic visualizations of a patient.

A final de-truncated volume may be generated based on blending de-truncated projections with truncated projections. For example, a de-truncated stack of projections may be blended with a truncated stack of projections using attenuation matching. By blending the two stacks of projections, the utilization of data inferred by the neural network may be reduced. As a result, the possibility of hallucinations may be reduced and/or identify instances of model hallucination that result in inaccurate corrections of the de-truncated volume.

A scatter correction model (e.g., Acuros) to correct the truncated stack of projections based on the de-truncated volume. By utilizing the scatter correction model, inaccuracies due to physical phenomena (e.g., scatter, beam hardening, and/or the like) may be reduced.

One or more recursive inferences may be added to the de-truncation workflow to further improve the de-truncated volume. By feeding a model prediction back into a model to cause it to generate an updated de-truncated volume, the accuracy of the de-truncated volume may be increased.

In one embodiment, a method comprises receiving, by at least one processor, a non-truncated volume including a three-dimensional representation of an anatomical region, the non-truncated volume representing imaging data within a full scan field of view (SFoV); simulating, by the at least one processor based on the non-truncated volume, an imaging system capturing a stack of projections including two-dimensional representations of the non-truncated volume, thereby generating a non-truncated stack of projections; simulating, by the at least one processor, truncation of the anatomical region by changing at least one visual attribute of the non-truncated stack of projections outside a predefined SFoV threshold, thereby generating a truncated stack of projections; reconstructing, by the at least one processor, a non-truncated training volume based on the non-truncated stack of projections and a truncated training volume based on the truncated stack of projections; forming, by the at least one processor, a set of training pairs including non-truncated training volumes paired with truncated training volumes, wherein the non-truncated volumes are designated as ground truth; and training, by the at least one processor, a machine learning model using the set of training pairs, wherein the machine learning model is configured to infer missing regions in truncated volumes.

Artificially simulating truncation may include trimming a portion of a projection in the non-truncated stack of projections.

Artificially simulating truncation may emulate an imaging apparatus not fully capturing the anatomical region.

The machine learning model may use a composite loss function including at least one of voxel-wise reconstruction loss, perceptual loss, or adversarial loss.

The non-truncated volume may be obtained using cone beam computed tomography (CBCT).

The plurality of training pairs within the set of training pairs may be generated based on a plurality of geometric transformations of the non-truncated volume, further including: transforming, by the at least one processor, the non-truncated volume in response to receiving the non-truncated volume by applying a geometric transformation to the non-truncated volume.

Training, by the at least one processor, may include: generating, by the at least one processor, a de-truncated volume based on providing a truncated volume from the set of training pairs to the machine learning model to cause the machine learning model to generate the de-truncated volume; and evaluating, by the at least one processor, whether data representing a portion of the de-truncated volume is associated with measured data obtained from an imaging system or associated with the inferred missing region.

The method may include executing, by the at least one processor, Hounsfield Unit matching between the de-truncated volume and the truncated training volume.

In another embodiment, a computer system may comprise a computer-readable medium storage including a set of non-transitory instructions, that when executed, cause a processor to: receive a non-truncated volume including a three-dimensional representation of an anatomical region, the non-truncated volume representing imaging data within a full scan field of view (SFoV); simulate an imaging system capturing a stack of projections including two-dimensional representations of the non-truncated volume, thereby generating a non-truncated stack of projections; simulate truncation of the anatomical region by changing at least one visual attribute of the non-truncated stack of projections outside a predefined SFoV threshold, thereby generating a truncated stack of projections; reconstruct a non-truncated training volume based on the non-truncated stack of projections and a truncated training volume based on the truncated stack of projections; form a set of training pairs including non-truncated training volumes paired with truncated training volumes, wherein the non-truncated volume is designated as ground truth; and train a machine learning model using the set of training pairs, wherein the machine learning model is configured to infer missing regions in truncated volumes.

The set of non-transitory instructions may further cause the processor to: generate a de-truncated volume based on providing a truncated volume from the set of training pairs to the machine learning model to cause the machine learning model to generate the de-truncated volume; and evaluate whether data representing a portion of the de-truncated volume is associated with measured data obtained from an imaging system or associated with the inferred missing region.

Artificially simulating truncation may include trimming a portion of the non-truncated stack of projections.

Artificially simulating truncation may emulate an imaging apparatus not fully capturing the anatomical region.

The machine learning model may use at least one of a composite loss function including voxel-wise reconstruction loss, perceptual loss, or adversarial loss.

The non-truncated volume may be obtained using cone beam computed tomography (CBCT).

A plurality of training pairs within the set of training pairs may be generated based on a plurality of geometric transformations of the non-truncated volume, and wherein the instructions further cause the processor to: transform the non-truncated volume in response to receiving the non-truncated volume by applying a geometric transformation to the non-truncated volume.

In another embodiment, a computer system comprises: a machine learning model; and a processor in communication with the machine learning model, the processor configured to: receive a non-truncated volume including a three-dimensional representation of an anatomical region, the non-truncated volume representing imaging data within a full scan field of view (SFoV); simulate an imaging system capturing a stack of projections including two-dimensional representations of the non-truncated volume, thereby generating a non-truncated stack of projections; simulate truncation of the anatomical region by changing at least one visual attribute of the non-truncated stack of projections outside a predefined SFoV threshold, thereby generating a truncated stack of projections; reconstruct a non-truncated training volume based on the non-truncated stack of projections and a truncated training volume based on the truncated stack of projections; form a set of training pairs including non-truncated training volumes paired with truncated training volumes, wherein the non-truncated volume is designated as ground truth; and train a machine learning model using the set of training pairs, wherein the machine learning model is configured to infer missing regions in truncated volumes.

The processor may be further configured to: generate a de-truncated volume based on providing a truncated volume from the set of training pairs to the machine learning model to cause the machine learning model to generate the de-truncated volume; and evaluate whether data representing a portion of the de-truncated volume is associated with measured data obtained from the imaging system or associated with the inferred missing region.

Artificially simulating truncation may include trimming a portion of the non-truncated stack of projections.

Artificially simulating truncation may emulate an imaging apparatus not fully capturing the anatomical region.

The machine learning model may use a composite loss function including at least one of voxel-wise reconstruction loss, perceptual loss, or adversarial loss.

In one embodiment, a method comprises receiving, by at least one processor, a truncated stack of projections including a set of two-dimensional representations of at least one anatomical structure of a patient, wherein the truncated stack of projections includes missing or incomplete data corresponds to one or more regions outside a scan field of view; reconstructing, by the at least one processor, a truncated volume including a three-dimensional representation of the at least one anatomical structure based on the truncated stack of projections; executing, by the at least one processor, a trained neural network model using the truncated volume, the trained neural network model being configured to infer missing regions within the truncated volume and to predict a de-truncated volume; simulating, by the at least one processor, an imaging system capturing a stack of projections including two-dimensional representations of the de-truncated volume, thereby generating a de-truncated stack of projections; blending, by the at least one processor, the de-truncated stack of projections with the truncated stack of projections to form a combined stack of projections; and reconstructing, by the at least one processor, a combined volume from the combined stack of projections using an iterative reconstruction algorithm (ART).

The method may further include simulating, by the at least one processor, at least one additional projection data beyond the scan field of view of the truncated volume.

The iterative reconstruction algorithm used for the combined volume may be an ART.

The neural network model may be trained on synthetic data pairs including truncated and non-truncated volumes.

The truncated stack of projections may be obtained using cone beam computed tomography (CBCT) imaging.

Blending the de-truncated stack of projections may include determining a modification of the de-truncated stack of projections based on performing attenuation matching between the de-truncated stack of projections and the truncated stack of projections.

The method may further include determining, by the at least one processor, a reliability of the de-truncated volume based on aleatoric or epistemic uncertainty.

In another embodiment, a computer system may comprise a computer-readable medium storage including a set of non-transitory instructions, that when executed, cause a processor to: receive a truncated stack of projections including a set of two-dimensional representations of at least one anatomical structure of a patient, wherein the truncated stack of projections includes missing or incomplete data corresponds to one or more regions outside a scan field of view; reconstruct a truncated volume including a three-dimensional representation of the at least one anatomical structure based on the truncated stack of projections; execute a trained neural network model using the truncated volume, the trained neural network model being configured to infer missing regions within the truncated volume and to predict a detruncated volume; simulate an imaging system capturing a stack of projections including two-dimensional representations of the de-truncated volume, thereby generating a de-truncated stack of projections; blend the de-truncated stack of projections with the truncated stack of projections to form a combined stack of projections; and reconstruct a combined volume from the combined stack of projections using an ART.

The set of non-transitory instructions may further cause the processor to simulate at least one additional projection data beyond the scan field of view of the truncated volume.

The iterative reconstruction algorithm used for the combined volume may be an ART.

The neural network model may be trained on synthetic data pairs including truncated and non-truncated volumes.

The truncated stack of projections may be obtained using cone beam computed tomography (CBCT).

The set of non-transitory instructions that cause the processor to blend the detruncated stack of projections with the truncated stack of projections may further cause the processor to determine a modification of the de-truncated stack of projections based on performing attenuation matching between the de-truncated stack of projections and the truncated stack of projections.

The set of non-transitory instructions may further cause the processor to determine a reliability of the de-truncated volume based on aleatoric or epistemic uncertainty.

In some aspects, a computer system comprises a medical imaging system; and a processor in communication with the medical imaging system, the processor configured to: receive, from the medical imaging system, a truncated stack of projections including a set of two-dimensional representations of at least one anatomical structure of a patient, wherein the truncated stack of projections includes missing or incomplete data corresponds to one or more regions outside a scan field of view; reconstruct a truncated volume including a three-dimensional representation of the at least one anatomical structure based on the truncated stack of projections; execute a trained neural network model using the truncated volume, the trained neural network model being configured to infer missing regions within the truncated volume and to predict a de-truncated volume; simulate an imaging system capturing a stack of projections including two-dimensional representations of the de-truncated volume, thereby generating a de-truncated stack of projections; blend the de-truncated stack of projections with the truncated stack of projections to form a combined stack of projections; and reconstruct a combined volume from the combined stack of projections using an ART.

The processor may be further configured to simulate at least one additional projection data beyond the scan field of view of the truncated volume.

The iterative reconstruction algorithm used for the combined volume may be an ART.

The neural network model may be trained on synthetic data pairs including truncated and non-truncated volumes.

The truncated volume may be scatter corrected.

The processor may be further configured to determine a reliability of the detruncated volume based on aleatoric or epistemic uncertainty.

### BRIEF DESCRIPTION OF THE DRAWINGS

**FIG. 1** illustrates components of an AI-enabled system for generating de-truncated volumes, according to an embodiment.
**FIG. 2** illustrates a flow diagram of a process for training a model to generate detruncated volumes, according to an embodiment.
**FIG. 3** illustrates a training data set used to execute deep learning for de-truncation of volumes, according to an embodiment.
**FIG. 4** shows an example environment for medical imaging and resulting image reconstructions, according to an embodiment.
**FIG. 5** illustrates an example of truncation, according to an embodiment.
**FIG. 6** illustrates a flow diagram of a process for generating de-truncated volumes, according to an embodiment.
**FIG. 7** illustrates an example of projection blending, according to an embodiment.
**FIG. 8** illustrates a graph of density values for different reconstructions of a stacks of projection, according to an embodiment.
**FIG. 9** illustrates reconstructions of a stack of projections using various reconstruction methods, according to an embodiment.
**FIG. 10** illustrates a depiction of aleatoric uncertainty, according to an embodiment.
**FIG. 11** illustrates a depiction of epistemic uncertainty, according to an embodiment.
**FIG. 12** illustrates a non-limiting example of a process for model training for generating de-truncated volumes, according to an embodiment.
**FIG. 13** illustrates a non-limiting example of a process for generating de-truncated volumes, according to an embodiment.
**FIG. 14** illustrates a non-limiting example of a process for generating de-truncated volumes with scatter-correction, according to an embodiment.

Non-limiting embodiments of the present disclosure are described by way of example with reference to the accompanying figures, which are schematic and are not intended to be drawn to scale. Unless indicated as representing the background art, the figures represent aspects of the disclosure.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are configured to be considered within the scope of the subject matter disclosed herein. Other embodiments can be used and/or other changes can be made without departing from the spirit or scope of the present disclosure. The illustrative embodiments described in the detailed description are not meant to be limiting of the subject matter presented.

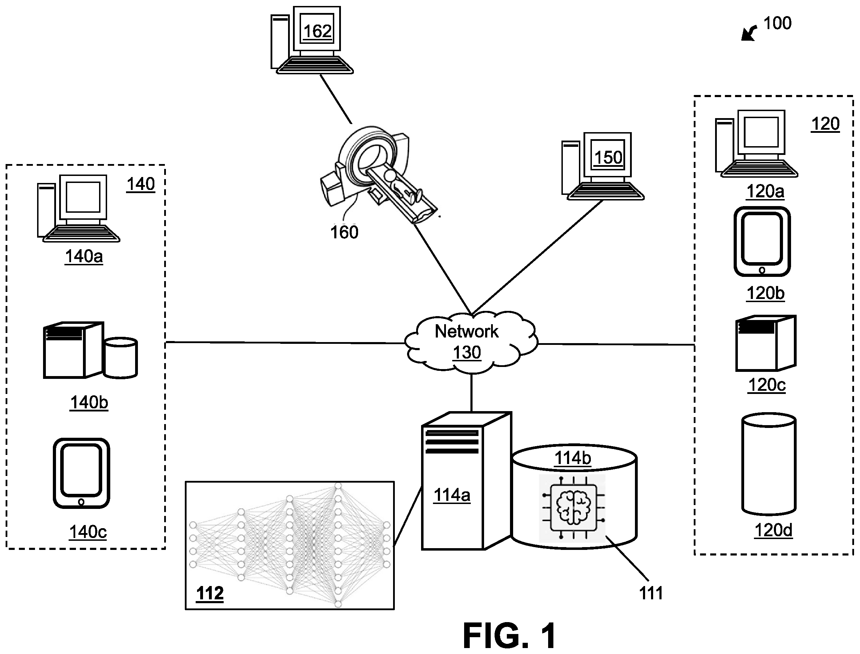

**FIG.** 1 illustrates components of an AI-enabled system for generating de-truncated volumes, according to an embodiment. The system **100** can include an analytics server **114a,** system database **114b,** a treatment planning system **111,** and electronic data sources **120a-d** (each referred to individually as an electronic data source **120** and collectively electronic data sources **120,** unless stated otherwise), end-user devices **140a-c** (each referred to individually as an end-user device **140** and collectively as end-user devices **140,** unless stated otherwise), an administrator computing device **150,** a medical device **160,** and medical device computer(s) **162.**

Various components depicted in **FIG. 1** can belong to a radiotherapy clinic at which patients can receive radiotherapy treatment, in some cases via one or more radiotherapy machines located within the clinic (e.g., medical device **160).** The system **100** is not confined to the components described herein and can include additional or other components, not shown for brevity, which are configured to be considered within the scope of the embodiments described herein.

The above-mentioned components can be connected to each other through a network **130.** Examples of the network **130** can include, but are not limited to, private or public local-area-networks (LAN), wireless LAN (WLAN) networks, metropolitan area networks (MAN), wide-area networks (WAN), and the Internet. The network **130** can include wired and/or wireless communications according to one or more standards and/or via one or more transport mediums. The communication over the network **130** can be performed in accordance with various communication protocols such as Transmission Control Protocol and Internet Protocol (TCP/IP), User Datagram Protocol (UDP), and IEEE communication protocols. In one example, the network **130** can include wireless communications according to Bluetooth specification sets or another standard or proprietary wireless communication protocol. In another example, the network **130** can also include communications over a cellular network, including, e.g., a GSM (Global System for Mobile Communications), CDMA (Code Division Multiple Access), and EDGE (Enhanced Data for Global Evolution) network.

The analytics server **114a** can be any computing device comprising a processor and non-transitory machine-readable storage capable of executing the various tasks and processes described herein. The analytics server **114a** can employ various processors such as central processing units (CPU) and graphics processing unit (GPU), among others. Non-limiting examples of such computing devices can include workstation computers, laptop computers, server computers, and the like. While the system **100** includes a single analytics server **114a,** the analytics server **114a** can include any number of computing devices operating in a distributed computing environment, such as a cloud environment.

The analytics server **114a** can generate and display an electronic platform configured to use a treatment planning system **111** for receiving patient information, inputs from users (e.g., clinicians) such as utility functions and updated utility functions described herein, and outputting the results of execution of the treatment planning system **111.** The electronic platform can include graphical user interfaces (GUI) displayed by display devices of one or more electronic data sources **120,** the end-user devices **140,** the medical device **160,** and/or the administrator computing device **150.** An example of the electronic platform generated and hosted by the analytics server **114a** can be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the treatment planning system **111.** For instance, the analytics server **114a** can execute the treatment planning system **111** (e.g., a system such as a treatment planner that is configured and/or trained to generate fluence maps, leaf sequences, etc., as described herein for a patient being treated via the medical device **160).** The analytics server **114a** can then display the results for a clinician and/or directly revise one or more operational attributes of the medical device **160.**

The electronic data sources **120** can be any computing device comprising a processor and non-transitory machine-readable storage capable of executing the various tasks and processes described herein. For example, the electronic data sources **120** can represent various computing devices that contain, retrieve, and/or access data associated with a medical device **160,** such as data associated with operational information of currently or previously performed radiotherapy treatments (e.g., electronic log files or electronic configuration files), data associated with current and/or previously monitored patients (e.g., computed tomography (CT) scans, magnetic resonance imaging (MRI) scans, tumor locations, deformation information, and/or the like) or participants in a study, and/or the like. For instance, the analytics server **114a** can use the clinic computer **120a,** medical professional device **120b,** server **120c** (associated with a clinician and/or a clinic), and database **120d** (associated with the clinician and/or the clinic) to retrieve/receive data associated with the medical device **160.** The analytics server **114a** can retrieve the data from the end-user devices **140,** generate a dataset, and use the dataset to configure the treatment planning system **111** (e.g., models implemented by the treatment planning system **111** and/or the like). The analytics server **114a** can execute various algorithms to translate raw data received/retrieved from the electronic data sources **120** into machine-readable patients that can be stored and processed by other analytical processes as described herein.

End-user devices **140** can be any computing device comprising a processor and a non-transitory machine-readable storage medium capable of performing the various tasks and processes described herein. Non-limiting examples of an end-user device **140** can be a workstation computer, laptop computer, tablet computer, or server computer. In operation, various users such as clinicians as described herein can use end-user devices **140** to access the GUI operationally managed by the analytics server **114a** or otherwise the results of the execution of the treatment planning system **111.** Specifically, the end-user devices **140** can include clinic computer **140a,** clinic server **140b,** and a medical professional device **140c.**

Even though referred to herein as "end-user" devices, these devices cannot always be operated by end-users. For instance, the clinic server **140b** cannot be directly used by an end user. However, the results stored on the clinic server **140b** can be used to populate various GUIs accessed by an end user via the medical professional device **140c.** In some embodiments, the end-user device **140** can be associated with one or more clinicians that are associated with the generation of one or more treatment plans (e.g., involved in preparing the one or more treatment plans) for patients.

The administrator computing device **150** can represent a computing device operated by a system administrator. The administrator computing device **150** can be configured to display radiotherapy treatment attributes generated by the analytics server **114a** (e.g., various analytic metrics determined during training of one or more machine learning models and/or systems); monitor various treatment planning systems **111** utilized by the analytics server **114a,** electronic data sources **120,** and/or end-user devices **140;** review feedback; and/or facilitate training or retraining (calibration) of the treatment planning system **111** that are maintained by the analytics server **114a.** In some embodiments, the methods and systems discussed herein may utilize transfer learning paradigms, such as by adapting a pre-trained machine learning model, originally trained on a broad dataset, using/leveraging the learned knowledge of the models discussed herein.

In some embodiments, the medical device **160** can be a diagnostic imaging device or a treatment delivery device. For example, the medical device **160** can include one or more computed tomography (CT) scanners, linear accelerators (LINACs) having a multi-leaf collimator (MLC) that consists of multiple small lead leaves that can be individually moved to shape the radiation beam and deliver the dose to the tumor while minimizing the dose to surrounding healthy tissues, or other similar devices configured to transmit energy toward targeted tissue (referred to as planning target volumes) associated with a patient and, in some cases, measure the energy transferred to ward the targeted tissue. The medical device **160** can also include one or more sensors configured to monitor the patient being treated. That is, the medical device **160** and/or the analytics server **114a** can be communicating with various sensors that can monitor a patient's external biological signals. Non-limiting examples of the sensors can include 3D surfacing mechanisms and optical (or other) sensors configured to monitor the patient's movements (e.g., how the patient is moving and/or breathing. In some embodiments, the medical device **160** can receive data associated with a treatment plan from the medical device computer(s) **162** that cause the medical device **160** to operate in accordance with the treatment plan.

The treatment planning system **111** can be stored in the system database **114b.** The treatment planning system **111** can be trained using data received/retrieved from the electronic data sources **120** and can be executed using data received from the end-user devices, the medical device **160.** In some embodiments, the treatment planning system **111** can reside within a data repository local or specific to a clinic. In various embodiments, the treatment planning system **111** can use one or more deep learning engines to develop a treatment plan for a patient using radiation therapy. For instance, the analytics server **114a** can transmit patient attributes and execute the treatment planning system **111** accordingly. The analytics server **114a** can then display the results on one or more end-user devices **140.** In some embodiments, the analytics server **114a** can change one or more configurations of the medical device **160** based on the results predicted by the treatment planning system **111.**

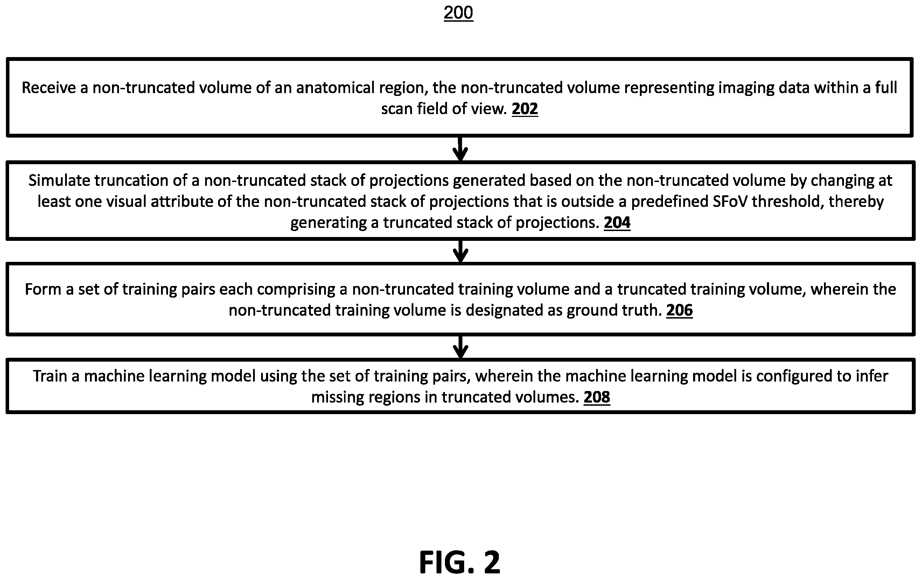

The system **100** may also include an artificial intelligence model **112.** The artificial intelligence model **112** may perform the image verification and analysis. In some embodiments, the artificial intelligence model **112** may be trained by the analytics server **114a.** For example, the artificial intelligence model **112** may be trained to de-truncate volumes by a method that is the same or similar to the process **200** of **FIG. 2****.** The artificial intelligence model **112** may be specifically designed and trained for the processing and analysis of medical imaging data. Initially, the artificial intelligence model **112** may engage in preprocessing the volumes, which involves refining image quality through adjustments in contrast, noise reduction, and standardization of size and resolution to ensure consistency across different imaging modalities. Following preprocessing, the artificial intelligence model **112** may utilize deep learning algorithms, particularly convolutional neural networks (CNNs), to extract key features from the images. These features may represent distinctive markers capable of accurately distinguishing one patient from another. After feature extraction, these characteristics are transformed into feature vectors. This transformation encodes the nuanced anatomical features into a numerical format, compactly representing each image's essential traits. In the latent space, vectors representing similar features may cluster together while those representing dissimilar features may be positioned apart. The artificial intelligence model **112** may calculate distances between feature vectors to estimate the similarity between corresponding features across different images, thus predicting the likelihood of images belonging to the same patient. In some embodiments, the artificial intelligence model **112** may use deep learning algorithms to predict missing portions of volumes. For example, the artificial intelligence model **112** may infer portions of a truncated volume that are missing from the associated stack of projections. The artificial intelligence model **112** may generate a detruncated volume wherein the missing portions are displayed based on the inference.

Finally, the artificial intelligence model **112** may interpret these analytical results to provide clinical decision support, e.g., by outputting a likelihood score or categorical data, which assists healthcare professionals in confirming patient identity or aiding in diagnostic decisions.

The artificial intelligence model's **112** adaptability allows for seamless integration with various medical imaging systems and electronic health records, enabling it to be trained on diverse datasets to accommodate different medical conditions and imaging techniques such as CT scans, MRI, X-rays, fluoroscopy, tomosyntheses, and/or ultrasound. Even though the artificial intelligence model **112** is depicted as a single model, it could be a collection of sub-models. Therefore, no limitation is intended.

### Training the machine learning model

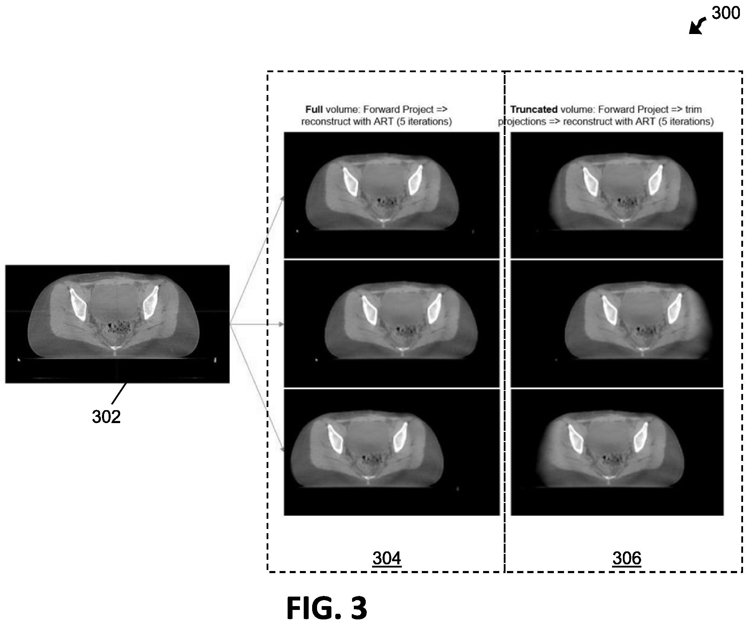

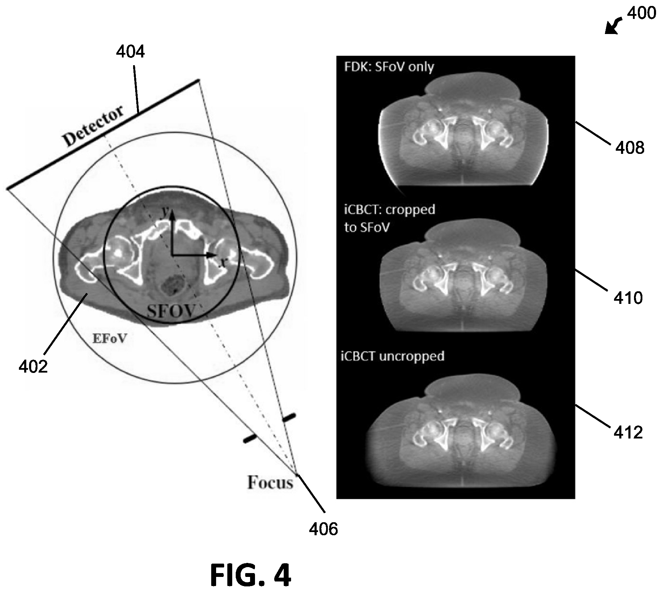

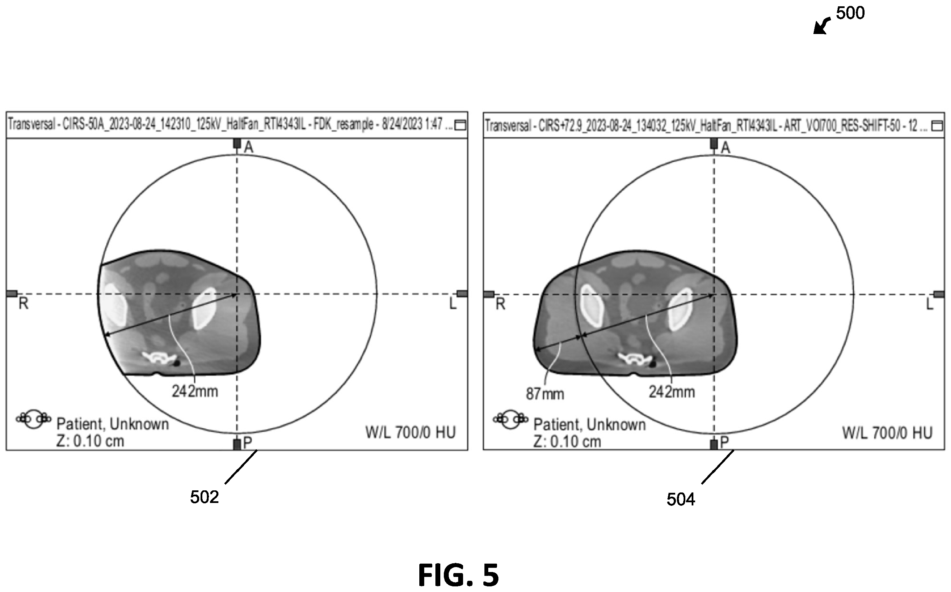

The analytics server may generate a training data set including pairs of simulated truncated volumes and non-truncated volumes. A machine learning model can then be trained using the curated training dataset to predict/generate de-truncated volumes based on the truncated volumes, and parameters of the machine learning model can be adjusted based on the de-truncated volumes and the non-truncated volumes. **FIG. 2** is a non-limiting example of a process for generating the training data set and training the machine learning model, **FIG. 3** depicts a non-limiting example of pairs within a training data set, and **FIG. 4** and **FIG. 5** depict non-limiting examples of truncation.

Referring to **FIG. 2****,** illustrated is a flow diagram of a process **200** for training a model to generate de-truncated volumes, according to an embodiment. The process **200** includes operations **202-208.** However, other embodiments can include additional or alternative operations or can omit one or more operations altogether. The process **200** is described as being executed by an analytics server, which can be the same as, or similar to, the analytics server **114a** described in **FIG. 1****.** However, one or more steps of the process **200** can be executed by any number of computing devices operating in the distributed computing system described in **FIG. 1****.** For instance, one or more computing devices can locally perform part or all of the operations described in **FIG. 2****.**

Computed Tomography (CT) scans can be used to visualize internal structures of a patient. CT scans can utilize cone-shaped X-ray beams (e.g., in Cone Beam CT (CBCT) systems) to generate three-dimensional representations of the patient. To generate representation of internal structures of a patient, an imaging system (e.g., imaging apparatus) can capture a stack of two-dimensional projections and reconstruct a three-dimensional representation of the patient (e.g., volume) based on the stack of projections. This may be useful for a wide range of medical purposes, such as radiotherapy treatment planning. For example, CT scans can be used to obtain information about a tumor within a patient. This information can be used to plan and monitor radiotherapy treatment plans, which require detailed information about the size and location of tumors to minimize the impact of radiation to healthy tissue and optimize radiation dosage to effectively target a tumor.

However, CT scans can sometimes be truncated due to a patient not being entirely within the scanned field of view (SFoV) of an imaging system. For example, a patient may be larger than the SFoV of the scanner or incorrectly positioned such that they are not entirely within the SFoV. As a result, the CT scanner may not capture all of the necessary information to generate a reconstruction of the internal structures of the patient. While some methods of reconstructing volumes representing internal structures of patients may be able to estimate the portion of the volume that is outside of the SFoV, this estimation may lack accuracy, particularly as distance from the edge of the SFoV increases. Furthermore, other methods may not be able to generate any reconstruction outside of the SFoV, resulting in volume that is cropped.

In examples where the reconstruction method estimates the portion of the volume that is outside of the SFoV, a machine learning model (e.g., neural network) can be used to correct the estimated portion of the volume based on training data including full volumes (e.g., the patient is entirely within the SFoV) and truncated volumes. However, training a machine learning model to perform this task requires large amounts of high-quality data including corresponding pairs of full and truncated volumes. It may be difficult to find existing pairs, since it may not be possible to obtain a full volume (e.g., because the patient is too large for the imaging system), or it may be discouraged to repeat CT scans to fix positioning issues because of radiation exposure associated with CT scans. To solve this lack of data, full volumes can be artificially truncated. However, to train the machine learning model to accurately correct truncated volumes, simulated truncated volumes may need to realistically simulate the estimate that would be generated for the section outside the SFoV. Furthermore, since training the machine learning model requires large datasets, and existing CT scans are typically stored and available in the form of three-dimensional volumes, systems and methods discussed herein may be needed for generating training pairs for the machine learning model based on full volumes.

The process **200** can simulate truncation to generate a training dataset that can be used to train a machine learning model to de-truncate volumes. The training dataset can include pairs of non-truncated and truncated volumes, which can be used to train the machine learning model to correct truncated volumes. In described examples, the imaging system can capture a stack of projections comprising a plurality of two-dimensional images taken from a variety of angles around the patient. For example, in a CBCT imaging system the two-dimensional images can be captured using a cone-shaped X-ray beam. These stacks of projections can then be reconstructed into three-dimensional volumes using various reconstruction algorithms. The process **200** can train a machine learning model to de-truncate three-dimensional volumes that are truncated.

At step **202,** the analytics server may receive a non-truncated volume representing an anatomical region. The non-truncated volume may be reconstructed based on imaging data of a patient that is within a SFoV of an imaging system. For example, the medical imaging system may capture a stack of projections including two-dimensional imaging data of the patient from several angles. This stack of projections can be reconstructed into the non-truncated volume using various reconstruction algorithms (e.g., Feldkamp-Davis-Kress (FDK), iterative reconstruction (ART), and/or the like). In an example, the non-truncated volume may include a plurality of Hounsfield Unit (HU) values (e.g., as the voxel values of the volume) that indicate density of the internal structures of the patient. In some examples the analytics server may generate a non-truncated stack of projections based on the non-truncated volume. For example, the analytics server may perform a forward projection on the non-truncated volume to simulate a non-truncated stack of projections that would be generated by an imaging system.

At step **204,** the analytics server may simulate truncation of the non-truncated stack of projections. For example, the analytics server may change at least one visual attribute of the non-truncated stack of projections outside a predefined SFoV threshold. In this example, changing at least one visual attribute can include modifying one or more pixel values in the non-truncated stack of projections. The analytics server may, for instance, change one or more pixel values in a projection to a pixel value associated with air. As a result, the projection may imitate a projection that does not capture part of the patient (e.g., a truncated projection from the truncated stack of projections).

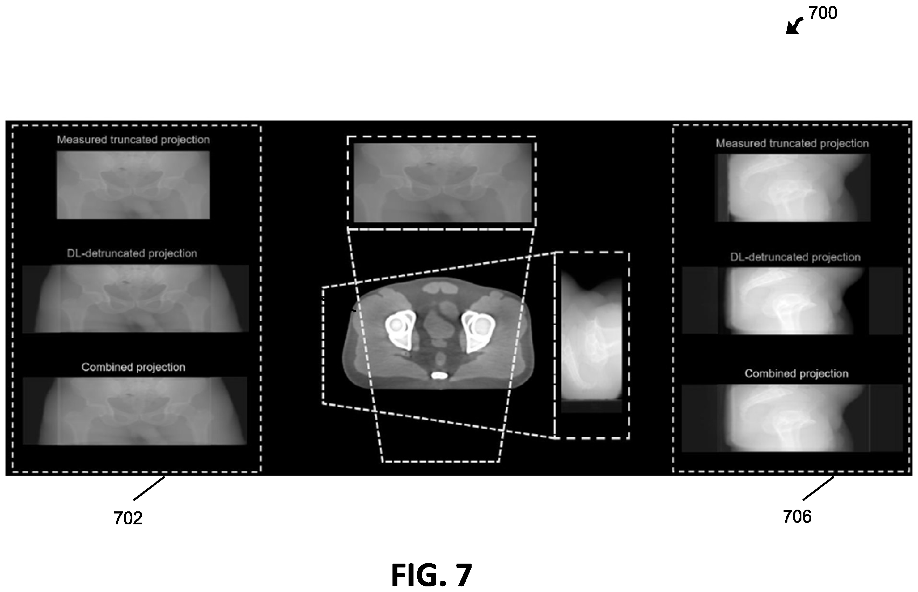

Based on parameters for an SFoV and/or EFoV, the analytics server can simulate truncation by removing (e.g., trimming) pixels from at least some of the projections. In an example, the analytics server may remove a portion of the patient that is in the EFoV for some projections. For example, the analytics server may simulate the view at each angle of a projection and remove portions of the projection accordingly. As an example, one projection in the stack of projections may be associated with a front-view of the patient (e.g., as depicted by the view associated with front-view projection blending **702** of **FIG. 7****),** and another projection in the stack of projections may be associated with a side-view of the patient (e.g., as depicted by the view associated with side-view projection blending **706** of **FIG. 7****).** In this example, the analytics server may remove pixels from the left and right side of the projection in the non-truncated stack of projections associated with the front-view (e.g., because part of the left and right side of the patient are outside the simulated view of the X-ray beam associated with the front-view projection) and/or may not remove pixels from the projection associated with the side-view (e.g., because the patient is entirely within the simulated view of the X-ray beam associated with the side-view projection). In some embodiments, analytics server can emulate a stack of projections from an imaging system (e.g., imaging apparatus) that does not fully capture the anatomical region. For example, the analytics server can trim the non-truncated stack of projections such that a certain portion of the patient (e.g., left shoulder, right side of the abdomen, and/or the like) is not present in the trimmed stack of projections.

In some embodiments, the analytics server may reconstruct a non-truncated training volume and a truncated training volume from the non-truncated stack of projections and the truncated stack of projections. For example, the analytics server may reconstruct these volumes using ART. The resulting truncated training volume can simulate a truncated volume resulting from a stack of projections captured by an imaging system (e.g., CT scanner) where the patient was not fully within the SFoV of the imaging system. The resulting non-truncated training volume can be similar to the non-truncated volume, however, the non-truncated training volume may reduce unintended variance between pairs of truncated and non-truncated training volumes used to train the machine learning model. In some examples, methods of generating a stack of projections (e.g., such as forward projection) may simplify some of the imaging data depicting the anatomical region. For example, the non-truncated stack of projections may not depict physical phenomena (e.g., noise, scatter, beam hardening, and/or the like). Training the machine learning model on the non-truncated training volume (e.g., as opposed to the non-truncated volume) may reduce discrepancies between truncated and non-truncated volumes that are not due to truncation.

At step **206,** the analytics server may form a set of training pairs including the non-truncated training volume and the truncated training volume. In this example, the non-truncated training volume may be designated as the ground truth. As such, the ground truth may serve as an ideal reconstruction that the machine learning model is trained to generate based on the truncated training volume.

In some embodiments, the analytics server may generate multiple training pairs in the set of training pairs based on one non-truncated volume. For example, the analytics server may perform a plurality of geometric transformations (e.g. shifting, rotating, flipping, scaling, elastic deformation, and/or the like) to the non-truncated volume. In this example, the analytics server may then perform forward projection on the transformed volumes to generate stacks of projections. As a result, the analytics server can augment the training data set with more training pairs, expose the machine learning model to a more diverse and comprehensive set of examples, which can lead to more reliable predictions.

At step **208,** the analytics server may train the machine learning model using the set of training pairs. For example, the machine learning model may generate a de-truncated volume based on the truncated training volume. Based on comparing the de-truncated volume to the non-truncated training volume, the analytics server may modify one or more parameters of the machine learning model (e.g., weights, biases, and/or the like). In some examples, training the machine learning model using volumes (e.g., as opposed to slices) can enforce longitudinal consistency. For example, a volume may be made up of a set of slices generated based on the stack of projections. Slices may represent cross-sectional images of the body, and a volume can be generated based on stacking the slices together. Generating predictions for each slice can result in inconsistencies along the stack of slices (e.g., longitudinally). This may result in artifacts when the volume is generated based on stacking the set of predicted slices together. Generating predictions based on projections may similarly result in artifacts within the final reconstructed volume, as predictions may be inconsistent between projections. As a result of generating de-truncated volumes based on executing a single prediction on truncated volumes, the machine learning model may reduce artifacts in the de-truncated volume that are due to inconsistent predictions between components (e.g., slices or projections) of the truncated volume.

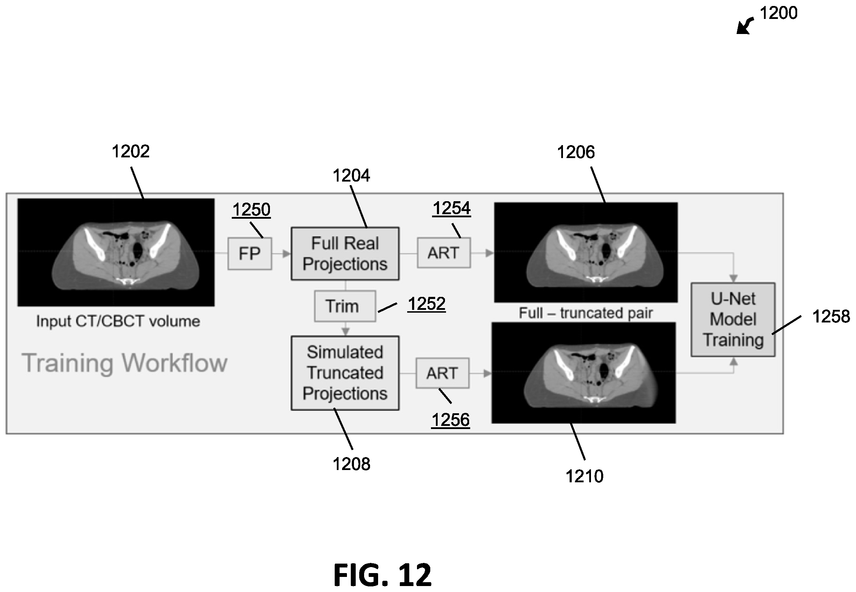

In some embodiments, the analytics server may train the machine learning model using a loss function. For example, the analytics server may compute a loss function corresponding to a difference between the predicted de-truncated volume and the non-truncated training volume used as the ground truth. In some examples, the loss function may include several differences. For example, the loss function may include voxel-wise loss (e.g., L1 loss as described in **FIG. 12****)** loss associated with features of the reconstruction (e.g., perceptual loss as described in **FIG. 12****),** or adversarial loss, as discussed below. In an example, the loss function may be a weighted composite of two or more types of loss.

In some embodiments, the machine learning may be trained using adversarial training. For example, the machine learning model may use generative adversarial network (GAN)-based architecture to generate a de-truncated volume based on the truncated training volume and then train the machine learning model based on non-truncated training volumes (e.g., ground truth volumes) and the generated volume. In an example, the analytics server may utilize a discriminator model to determine whether a portion of the de-truncated volume is generated by the machine learning model or captured by an imaging system. In this example, the discriminator model may evaluate whether a portion of the output of the machine learning model is generated by the model or associated with the non-truncated training volume. For example, the discriminator can be trained to identify inconsistent or unrealistic elements within the volume that indicate a flawed prediction (e.g., de-truncated volume) by the machine learning model. The discriminator may be trained, alongside the machine learning model (e.g., generator), based on whether it correctly or incorrectly identifying the portion of the detruncated volume. As a result, the machine learning model may be trained to generate data that is more similar to data generated by an imaging system (e.g., more realistic). In an embodiment, the machine learning model and/or discriminator model may be trained using loss functions. In this embodiment, the machine learning model may be trained to minimize the correct identification of generated data from the model and data in the truncated volume reconstructed from the truncated stack of projections generated by an imaging system (e.g., by an equation that is the same or similar to the generator loss equation described in **FIG. 12****).** Similarly, the discriminator model may be trained to maximize its classification accuracy (e.g., by an equation that is the same or similar to the discriminator loss equation described in **FIG. 12****).**

In some examples, the discriminator may identify a volume as a de-truncated volume from the generator based on identifying inconsistencies within a volume. For example, the discriminator can identify a mismatch of HU values in a volume, indicating inconsistent densities within the volume. This can occur as a result of an imperfect prediction of the machine learning model at the edge of the truncated volume. For example, the machine learning model may predict an extension of a structure within the truncated volume that does not match the density of the structure. In some examples, the discriminator can identify a volume as a de-truncated volume generated by the machine learning model based on this inconsistency.

In some embodiments, the analytics server may perform HU unit matching between the de-truncated volume and the non-truncated training volume. For example, the analytics server may determine if pixels (e.g., a single pixel, combination of pixels, and/or the like) differ by a threshold amount between the de-truncated volume and the non-truncated volume. In an example, the analytics server may perform HU matching between a portion of the de-truncated volume and the corresponding portion of the non-truncated training volume. In some examples, HU matching can be performed in addition to training the model (e.g., using the loss function). For example, it can be used to identify large differences between the designated ground truth and the prediction of the machine learning model. These differences can be used to identify faulty predictions, such as hallucinations.

Referring to **FIG. 3****,** illustrated is a training data set **300** used to execute deep learning for de-truncation of volumes, according to an embodiment. The training data set **300** comprises a set of full volumes **304** and a set of truncated volumes **306** generated based on an input volume **302.** In some examples, the input volume **302** may be a volume that has been reconstructed based on a stack of projections where the entire patient is within the SFoV of the imaging system that captured the stack of projections. In some implementations, multiple training pairs of a full volume and a corresponding truncated volume may be generated based on the input volume **302.** In the illustrated embodiment, transformations may be applied to the input volume **302.** As an example, three volumes where the first has no transformation, the second is shifted to the right, and the third is shifted to the left may be generated. In this example, the three full volumes **304** and corresponding truncated volumes **306** may be generated based on the three volumes generated from transformations of the input volume **302.** The full volumes **304** and the truncated volumes **306** may be generated by a process that is the same or similar to the training data set generation process described above. In some implementations, different stacks of simulated projections may be trimmed in different places. The stack of simulated projections corresponding to the first truncated volume in the truncated volumes **306** are trimmed on the left side (e.g., viewer's left side), the stack of simulated projections corresponding to the second truncated volume in the truncated volumes **306** are trimmed on the right side, and the stack of simulated projections corresponding to the third truncated volume in the truncated volumes **306** are trimmed on the left side. In examples, the stack of simulated projections and their corresponding trimmed stacks of simulated projections may be reconstructed using various methods. In the embodiment depicted by the full volumes **304** and the truncated volumes **306,** they are reconstructed using an iterative ART process with multiple iterations.

**FIG. 4** shows an example environment **400** for medical imaging and resulting image reconstructions, according to an embodiment. In the example environment **400,** a patient **402** may be scanned by an imaging system comprising a transmitter **406** and a detector **404.** The transmitter **406** may transmit a signal (e.g., X-Ray signal, and/or the like) through the patient **402** to the detector **404.** In some examples, the signal may spread out from a point source (e.g., a cone beam for Cone-Beam Computed Tomography (CBCT)). In examples where the signal spreads out from a point source, the detector **404** may be a flat panel that is larger than the transmitter **406.** Based on the received signal, the detector **404** may generate a two-dimensional (2D) projection illustrating the variations in intensity of the received signal. In some examples, the transmitter **406** and the detector **404** may rotate around the patient **402.** In these examples, the transmitter **406** and the detector **404** may rotate about the center of the patient **402.** As a result, the detector **404** may generate a stack of 2D projections of the patient **402** taken from various angles around the patient **402.** In some embodiments, a three-dimensional (3D) representation (e.g., volume) of the internal structure of the patient **402** may be generated based on the stack of projections. For example, the stack of projections may be reconstructed to obtain a volume representing the patient **402.** In some examples, the internal structure of the patient **402** obtained from the scan may be used for medical purposes, such as radiotherapy treatment planning. While embodiments discussed herein describe a patient being scanned for medical applications, it should be noted that the systems and methods may be used for other applications. For example, an animal may be scanned instead of a patient for medical applications. As another example, an object may be scanned instead of a person for industrial applications. Therefore, no limitation is intended.

An imaging system may have a scanned field of view (SFoV), where the field is within view of the imaging system at all angles, and an extended field of view (EFoV), where the field is in view of the imaging system at some, but not all, angles. In some embodiments, the patient **402** may only be within the (SFoV) at certain positions of the transmitter **406** and the detector **404.** As a result, some projections generated by the imaging system may be incomplete. As depicted in **FIG. 4****,** part of the patient's hips are not within the SFOV at that particular position of the transmitter **406** and the detector **404.** The entirety of patient **402** is within the extended field of view (EFoV), therefore all portions of the patient **402** may be captured on at least some of the projections within the stack of projections. However, since a portion of the patient **402** is not within the SFoV and therefore not present in every projection, the stack of projections may not capture all of the angles required to reconstruct a 3D projection of the portion of the patient **402** outside of the SFoV.

Various reconstruction techniques may be used to reconstruct a 3D projection from stacks if projections generated by an imaging system. In some embodiments, iterative reconstruction (e.g., algebraic reconstruction technique (ART)) may be utilized to construct a 3D volume from a stack of 2D projections. ART may be utilized to reconstruct the 3D volume from the stack of 2D projections by generating an initial volume estimate and iteratively adjusting this volume estimate multiple times to minimize the difference between measured data (e.g., the stack of projections) and estimated data (e.g., the generated volume). However, for angles at which a portion of the patient **402** is outside the SFoV, there may not be measured data to adjust the volume estimate on. While, in some implementations, ART can provide an estimation of intensity values in the EFoV based on incomplete measured data from some angles, the accuracy of this estimation quickly decreases as the distance from the SFoV boundary increases. Alternatively, a Feldkamp-Davis-Kress (FDK) algorithm may be utilized to construct a 3D volume from the stack of 2D projections. An FDK algorithm may be used to generate a 3D volume based on the stack of projections using a combination of filtering (e.g., by applying a convolution operation to emphasize high frequency details and correct for distortion) and back-projection (e.g., by distributing filtered projection data into a 3D volume). However, in standard implementations of FDK, the voxel values located outside of the SFoV are undefined. For example, values in the stack of projections that are outside of the SFoV may be removed, since incomplete data may result in artifacts and/or incorrect reconstruction. As represented in the FDK reconstruction **408,** an artifact comprising a white line is present at the line of truncation. This artifact may be a result of incomplete data outside if the SFoV in the stack of projections.

Depicted in **FIG. 4****,** as an example of different re-construction techniques, are three 3D reconstructions of the patient **402** based on the stack of projections captured by the imaging system. In the FDK reconstruction **408,** FDK is used to reconstruct the volume. In some examples, the FDK reconstruction **408** may be generated based only on the portion of the stack of projections that is within the SFoV. As a result, the reconstruction may not display any voxel values outside of the SFoV. In the cropped ART reconstruction **410,** an iterative reconstruction technique (e.g., ART) is used to reconstruct this volume. In this reconstruction, the volume is cropped to the SFoV. In some examples, the cropped ART reconstruction **410** may be a version of the ART reconstruction **412** where values outside of the SFoV have been removed. The ART reconstruction **412** displays a 3D reconstruction of the volume where the voxel values outside of the SFOV are an estimation of the intensity values. However, as discussed above, the accuracy of this estimation decreases as distance from the SFoV boundary increases. This can be observed in the ART reconstruction **412,** as the boundary of portion of the patient that is outside of the SFoV (e.g., the left and right side) is blurrier than the boundary of the patient that is within the SFoV (e.g., the front and back of the patient).

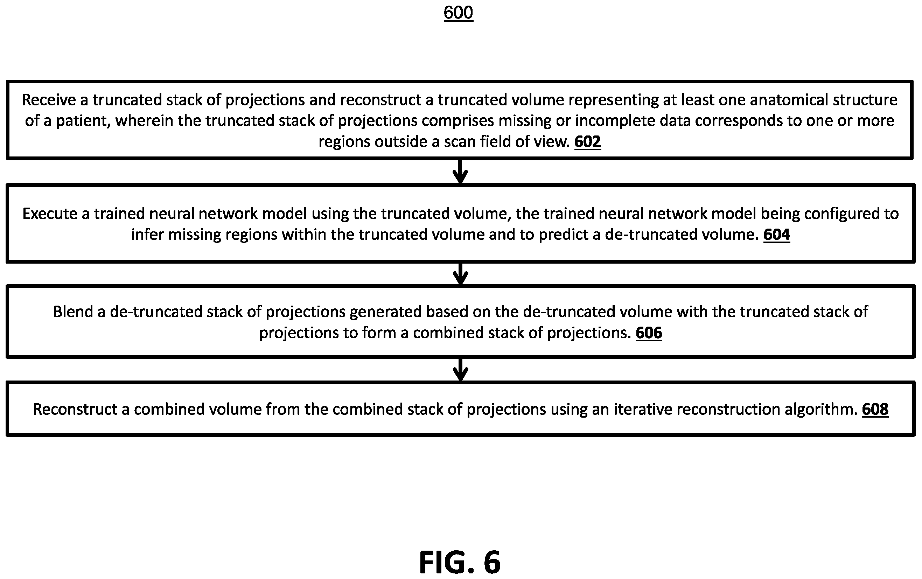

As described herein, an extended field of view deep learning (EFOV-DL) (e.g., that is the same or similar to the process **600** of **FIG. 6****)** technique may be used to reconstruct truncated stacks of projections. As part of this technique, a deep learning model may be utilized to correct a volume generated using an iterative reconstruction algorithm (e.g., ART). This technique may, for example, be used to de-truncate cone-beam CT images with sinogram recombination. A model (e.g., neural network, and/or the like) may be trained on a simulated training data set (e.g., at step **208** of **FIG. 2****).** In some implementations, the model may then be used to generate de-truncated projections. In these implementations, the model may generate a de-truncated 3D volume based on a truncated 3D volume that has been generated based on the truncated stack of projections. By virtue of utilizing the model, voxel values that are outside of the SFoV, and therefore lack sufficient information to be accurately generated by conventional reconstruction methods, may be predicted. As a result, the model may generate a more complete and/or accurate 3D volume than conventional methods of reconstruction.

Referring to **FIG. 5****,** illustrated is a pair of images **500** associated with a patient that is partially within the SFoV of an imaging system. Included in the pair of images is a reconstruction **502** of the patient with the SFoV represented as a circle and a representation **504** of the position of the patient within the SFoV. In some examples, the patient may be partially within the SFoV. As illustrated in the representation **504,** the SFoV has a radius of 242 mm, and the patient is 87 mm outside of the SFoV at the farthest point. In some examples, the portion of the patient that is outside of the SFoV (e.g., the circle) may still be within the EFoV. As a result, this portion of the patient may be present on some, but not all, scans in a stack of projections. The reconstruction **502** may be a reconstruction of a patient (e.g., the patient **402** of **FIG. 4****).** In some examples, the reconstruction **502** may be a FDK reconstruction. As a result, the values representing the portion of the patient that is outside of the SFoV in the reconstruction **502** may be undefined. This is displayed in the reconstruction **502** by the absence of the patient outside of the SFoV. The reconstruction **502** also displays an artifact comprising a white line along the truncation border (e.g., edge of the SFoV) that may be present in some FDK reconstructions due to truncation of stacks of projections.

### Executing the trained neural network

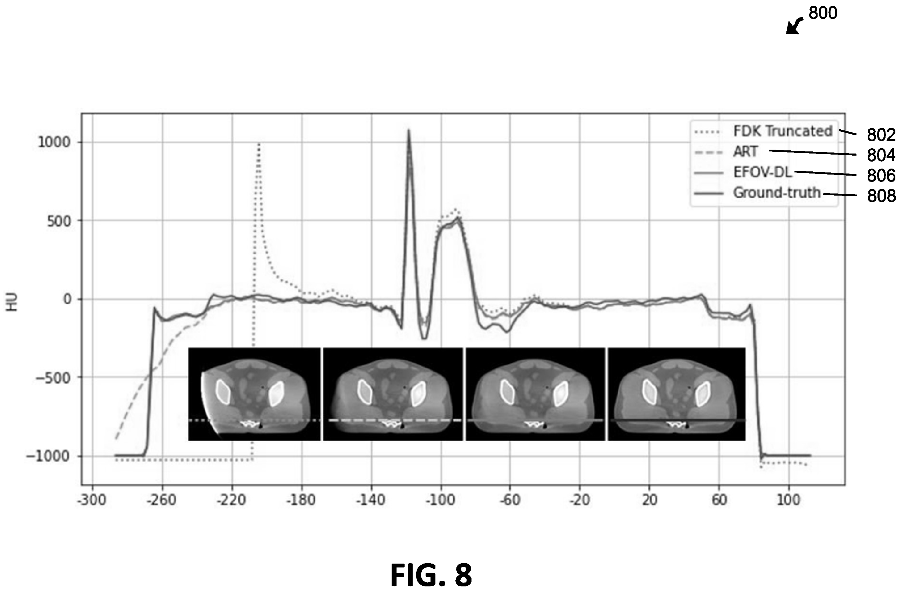

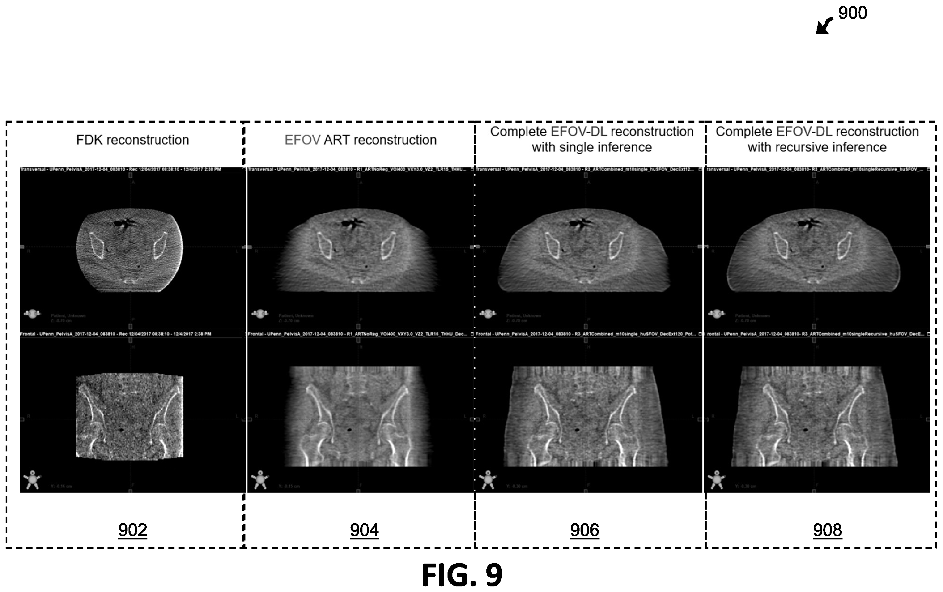

The machine learning model trained by the process **200** of **FIG. 2** may be a trained neural network. The analytics server can execute this trained neural network on truncated volumes to generate de-truncated volumes. As described in **FIG. 2****,** CT scans that are used for various medical purposes may sometimes generate truncated stacks of projections due a patient not being fully within an SFoV (e.g., due to size or position). Reconstruction methods such as ART can provide estimations for values outside the SFoV when generating volumes based on truncated stacks of projections, however, these estimates can lack accuracy. The steps described in **FIG. 6** can be used to execute a trained neural network to correct estimated voxel values in a volume generated based on a truncated stack of projections. **FIG. 7** depicts a non-limiting example of different projections views and projection blending. **FIG. 8** an **FIG. 9** depict different examples of reconstruction methods.

Referring to **FIG. 6** illustrated is a flow diagram of a process for generating detruncated volumes, according to an embodiment. The process **600** includes operations **602-608.** However, other embodiments can include additional or alternative operations or can omit one or more operations altogether. The process **600** is described as being executed by an analytics server, which can be the same as, or similar to, the analytics server **114a** described in **FIG. 1****.** However, one or more steps of the process **600** can be executed by any number of computing devices operating in the distributed computing system described in **FIG. 1****.** For instance, one or more computing devices can locally perform part or all of the operations described in **FIG. 6****.**

In examples where the reconstruction method (e.g., ART) generates an estimate for voxel values in the portion of the volume that is outside of the SFoV, a neural network can be used to correct the estimated portion of the volume based on training data including full volumes (e.g., the patient is entirely within the SFoV) and truncated volumes. Anatomical structures exhibit consistent patterns and predictable features, making them well-suited for accurate recreation and modeling by neural networks. However, model generated data may not be perfect and can exhibit flaws such as hallucination. Blending the de-truncated imaging data with truncated imaging data may mitigate possible errors due to these flaws. This method can allow model generated data to only be used for portions of the de-truncated volume that are missing in the truncated volume. As a result, described systems and methods can de-truncate a truncated volume while minimizing possible flaws resulting from imperfect data generated by the model.

Using the process **600,** the analytics server may generate a de-truncated volume based on generating a prediction of a de-truncated volume and blending the de-truncated volume with the truncated volume. In described examples, the imaging system (e.g., CBCT system) can capture a stack of projections comprising a plurality of two-dimensional images taken from a variety of angles around the patient. These stacks of projects can be reconstructed into three-dimensional volumes using various reconstruction algorithms.

At step **602,** the analytics server may receive a truncated stack of projections. For example, the truncated stack of projections may include two-dimensional representations (e.g., projections) of an anatomical structure of a patient (e.g., the abdomen of the patient) captured at various angles around the patient. In this example, the truncated stack of projections may be the result of a scan where part of the patient is outside the SFoV of an imaging system. For example, an imaging system may have an SFoV, where objects are captured in each projection, and an EFoV, where objects are only captured in some projections. In some examples, the patient may be too large to fit entirely within the SFoV or may be incorrectly positioned such that they are not entirely within the SFoV (e.g., when the patient **402** is larger than the SFoV depicted in **FIG. 4****).** As a result of the patient not being entirely within the SFoV, the imaging system may generate a truncated stack of projection that does not capture all of the necessary angles of the patient to reconstruct a complete volume. In some examples, the analytics server may reconstruct a truncated volume based on the truncated stack of projections. In an embodiment, the analytics server may reconstruct the volume using ART. In this embodiment, the truncated volume may include a partial estimate for portions of the patient that are outside of the SFoV. For instance, estimates may be generating using ART for voxel values that lack complete data in the truncated stack of projections.

At step **604,** the analytics server may execute the trained neural network model to infer missing regions of the truncated volume. Missing regions may refer to portions of the truncated volume that are not present or were reconstructed based on incomplete data. In an example, the analytics server may generate a de-truncated that modifies one or more voxel values of the truncated volume.

In some embodiments, the trained neural network model may be trained on synthetic data pairs comprising truncated and non-truncated volumes. For example, the trained neural network model may be trained by a process that is the same or similar to the process **200** of **FIG. 2****.** In some embodiments, the trained neural network model may be a U-Net convolutional neural network model. The U-Net convolutional neural network may include an encoder and a decoder and may downsample volumes with the encoder to capture high-level details and upsample volumes with the decoder to capture low-level features. In an example, the trained neural network model may be a standard U-Net model with attention gates incorporated, which can enhance sensitivity and prediction accuracy of the U-Net model.

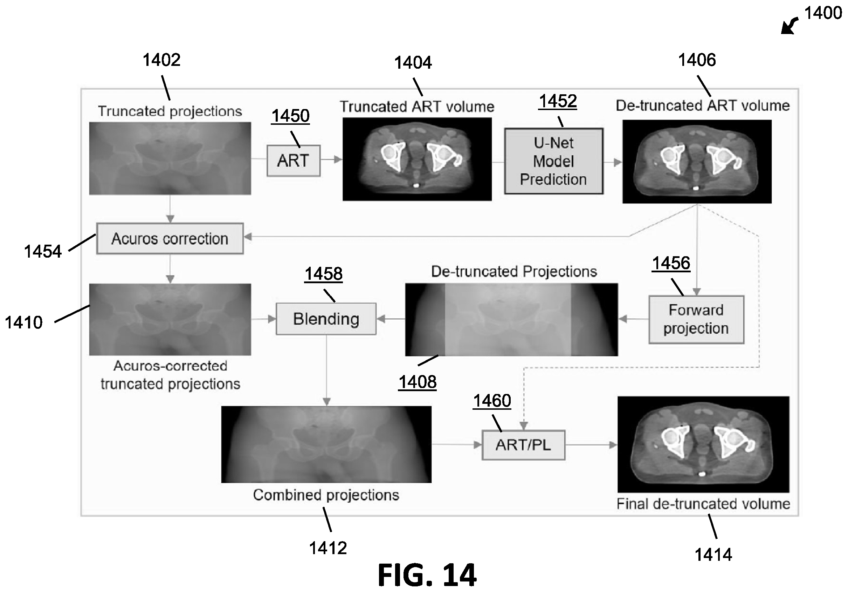

At step **606,** the analytics server may blend the de-truncated volume with the truncated volume. In an embodiment, the analytics server generates a de-truncated stack of projections based on the de-truncated volume. In this embodiment, the analytics server can simulate (e.g., using forward projection) a stack of projections based on the de-truncated volume. In this example, the analytics server may generate a combined stack of projections based on blending the de-truncated stack of projections with the truncated stack of projections. In some examples, this may minimize the utilization of inferred data in the de-truncated stack of projections. For example, the de-truncated and truncated stacks of projections may be blended to minimize the utilization of the de-truncated stack of projections (e.g., inferred data generated by the trained neural network model) within a portion of the blended stack of projections that corresponds to the SFoV. In this example, the analytics server may utilize data from the de-truncated stack of projections in portions of the blended stack of projections that are outside of the SFoV and data from the truncated stack of projections in portions of the blended stack of projections that are within the SFoV. In an example, the analytics server may use techniques such as linear ramping at borders between data from a projection in the detruncated stack of projections and data from a projection in the truncated stack of projections to improve blending of the border between the truncated and de-truncated stacks of projections. In some embodiments, the analytics server may implement scatter-correction as part of the process **600.** For example, the analytics server may implement a scatter-correction model in a method that is the same or similar to the scatter-corrected de-truncation workflow **1400** of **FIG. 14****.**

In some embodiments, the analytics server may perform attenuation matching as part of blending the truncated stack of projections with the de-truncated stack of projections. Attenuation matching can be used to modify the de-truncated stack of projections before they are blended with the truncated stack of projections. In an example, the analytics server may determine attenuation values of the truncated stack of projections and the de-truncated stack of projections. The analytics server may then compare attenuation values of corresponding projections in the truncated stack of projections and the de-truncated stack of projections. In this example, attenuation values may be compared within a region of interest. For example, the region of interest may be a certain number of columns next to the border between the truncated and de-truncated projections. The analytics server may determine mean attenuation values in this region of interest for each projection and then compare corresponding projections between the truncate and de-truncated stack of projections. Based on this comparison, the analytics server can determine a corrective offset (e.g., modification) for the de-truncated stack of projections. This corrective offset can then be applied to the de-truncated stack of projections prior to blending.

At step **608,** the analytics server may reconstruct a combined volume. For example, the analytics server may reconstruct the combined stack of projections into the combined volume. In an example, the analytics server may reconstruct the combined stack of projections using an iterative reconstruction technique such as ART.

In some embodiments, the analytics server may determine a reliability of the detruncated volume. For example, the analytics server can compute aleatoric uncertainty representing randomness of data. As another example, the analytics server can compute epistemic uncertainty representing uncertainty within model parameters of the trained neural network. In some examples, the analytics server may determine a reliability score based on aleatoric and/or epistemic uncertainty. The reliability score can represent a confidence in the reliability of predictions made by the model.

Referring to **FIG. 7****,** illustrated is an example of projection blending **700,** according to an embodiment. Depicted in this figure is an example of front-view projection blending **702** associated with a front view of a patient, a representation of the patient **704** and the positions of the projections relative to the patient, and an example of side-view projection blending **706** associated with a side view of the patient. With reference to the example of front-view projection blending **702,** depicted is a "Measured truncated projection", a "DL-detruncated projection" generated by a model (e.g., that is the same or similar to the neural network model of **FIG. 6****),** and a "Combined projection" generated based on the "Measured truncated projection" and the "DL-detruncated projection". As can be observed in the representation of the patient **704,** only part of the patient is within the SFoV of the front view, which is indicated by the dashed lines. As a result, the "Measured truncated projection" for the front view may not include a full view of the patient. In this example, the combined projection may primarily include data generated by the model to represent the portion of the patient that is outside of the SFoV. For example, the analytics server may use no data from the de-truncated projection inside the SFoV or minimal data (e.g., to ensure a smooth border between the truncated and de-truncated projection) to generate the combined projection. As a result, the analytics server may minimize possible flaws (e.g., artifacts, hallucinations, and/or the like) resulting from inaccurate data generated by the model. By contrast, the patient is fully within the SFoV of the side view, as can be observed in the representation of the patient **704.** In this example, the analytics server may not utilize data generated by the model to generate the combined projection based on determining that the patient is fully within the SFoV in this projection.

In some embodiments, the analytics server may perform attenuation matching to ensure compatibility between a pair of de-truncated and truncated projections. In an example, attenuation matching can account for discrepancies between the truncated and de-truncated projections. For example, the truncated and de-truncated projections can have discrepancies because the model may not generate predictions that simulate physical phenomena, such as scatter. In these embodiments, a region of interest may be selected between the pair of detruncated and truncated projections. For example, the region of interest may be a part of the truncated projection and the corresponding part of the de-truncated projection. As an example, the region of interest may be a preset number of columns on the left and/or right side of the truncated projection. In some implementations, the analytics server may compute mean attenuation values for the region of interest in both the truncated projection and the detruncated projection. Based on the difference between the mean attenuation values, the analytics server may then generate a corrective offset. In some examples, this corrective offset may be applied to the de-truncated projection. In these examples, this may result in the detruncated projection more closely matching the corresponding truncated projection. This may, for instance, result in a more seamless integration of the truncated and de-truncated projections. Since discrepancies in the combined projection may lead to artifacts in a resulting volume, correction based on attenuation matching may reduce the number of artifacts in a volume reconstructed from the combined projections. As another example, attenuation matching can ensure that physical areas (e.g., areas tissue, fat, and/or the like) have the same level of attenuation. For example, the attenuation levels of structures in the de-truncated projections can be corrected to match corresponding structures in the attenuation levels of the truncated projections. In an embodiment, the analytics server may perform correction based on attenuation matching with each pair of projections in the de-truncated and truncated projections. In an example where the correction is applied to the de-truncated projection, the region of interest from the de-truncated projection may be present (e.g., either as the sole values or as part of a linearly ramped region) in the resulting combined projection. In this example, the region of interest from the truncated projection may be present in the resulting combine projection.

In some examples, the analytics server may combine the truncated and de-truncated projections based on linear ramping. For example, a linear ramp function may be applied to a region of interest between a pair of de-truncated and truncated volumes to gradually shift from one projection to the other. In an example, the linear ramping along a barrier between a pair of truncated and de-truncated projections. As an example, the linear ramping may be performed on a certain number of columns on the left and/or right side of the "Measured truncated projection". The linear ramping may gradually shift the pixels from de-truncated pixels of the de-truncated projection to truncated pixels of the truncated projection on the left side of the "Measured truncated projection". In an example, this region may also be the region used for attenuation matching, as described above. For example, linear ramping may be applied to blend the region of interest between the truncated and de-truncated projections. The region of interest may, for instance, represent a region within the SFoV that is along the border with the EFoV. Attenuation mapping may be applied to the region of interest of the de-truncated projection to ensure proper blending with the truncated projection.

In some embodiments, the analytics server may re-train the model based on determining that the de-truncated stack of projections deviates by a threshold amount from the truncated stack of projections. For example, the analytics server may determine a numerical difference (e.g., by pixel-wise difference such as mean squared error, structural similarity index measure (SSIM), feature-based comparison, and/or the like) between corresponding projections in the truncated stack of projections and de-truncated stack of projections. In this example, the analytics server may not use extended portions (e.g., columns and/or rows that have been added to the projection in the forward projection process) of the de-truncated stack of projections in the comparison. In some embodiments, the analytics server may re-train the model based on determining that a single pair of projections in the truncated and de-truncated stacks deviate by a threshold amount. Additionally or alternatively, the analytics server may re-train the model based on determining that a combination (e.g., average, weighted average, and/or the like) of distances between pairs of projections in the truncated and de-truncated stacks of projections deviates by a threshold amount.

Referring to **FIG. 8****,** illustrated is a graph **800** of density values for different reconstructions of a stack of projections, according to an embodiment. Displayed is an FDK truncated reconstruction, ART reconstruction, and EFoV-DL (e.g., that is generated based on a process that is the same or similar to the process **600** of **FIG. 6****)** reconstruction of a patient from a stack of projections. The stack of projections is truncated on the left side of the patient. Additionally displayed, is a ground-truth reconstruction of the patient from a full (e.g., the patient is fully within the SFoV) stack of projections. This ground-truth represents an ideal reconstruction. Along the y-axis of the graph, the density of the voxels in the reconstruction is represented in HU. Along the x-axis of the graph, positions along profiles associated with the reconstructions of are displayed in millimeters. It should be noted that while the reconstructions are 3D representations of the patient, the plotted lines compare the density of the patient in a profile of the patient. The profile may be a one-dimensional representation of the variations in density along a specific line within a volume. This line is illustrated in each reconstruction displayed on the graph.

An indication of the accuracy of each reconstruction can be determined based the distance between the plotted line for the reconstructions associated with the FDK truncated line **802,** ART line **804,** and EFoV-DL line **806,** and the ground truth reconstruction associated with the ground-truth line **808.** As an example, the trajectory of the FDK truncated line **802** illustrates how the FDK reconstruction differs from the ground truth reconstruction because of the truncation present in the volume between the "-220" and "-180" mm positions on the x-axis. The trajectory of the ART line **804** shows how the estimation of intensity values for voxels within the EFoV results in a reconstruction that is closer to the ground truth than the FDK truncated line **802.** However, the EFoV-DL line **806** follows the ground-truth line **808** much more closely than both the FDK truncated line **802** and the ART line **804,** since the distance between the EFoV-DL line **806** and the ground-truth line **808** is much smaller than the distance between the ground-truth line **808** and the other two lines. This indicates that the EFoV-DL reconstruction may be a more accurate reconstruction of the patient.

Referring to **FIG. 9****,** illustrated are reconstructions **900** of a stack of projections using various reconstruction methods, according to an embodiment. In an embodiment, an imaging system (e.g., such as the imaging system of **FIG. 4****)** may capture a stack of projections representing a patient. Based on the stack of projections, a volume displaying the internal structure of the patient may be displayed. However, part of the patient may not be within the SFoV of the imaging system, resulting in truncated stack of projections. As a result, the stack of projections may not have enough information to reconstruct the portion of the patient that is not within the SFoV using some reconstruction methods. Illustrated in **FIG. 9** are four volumes that are generated based on a truncated stack of projections, where two views are displayed for each volume. The top row of views displays a view of the top of the patient, while the bottom row of views displays a view of the front of the patient. The orientations of the two views are further described by the "R" (e.g., right side), "L" (e.g., left side), "A" (e.g., anterior), "P" (e.g., posterior), "F" (e.g., foot), and "H" (e.g., head) markings describing the orientation of the patient on the eight views. These reconstructions may be generated by the analytics server **114a** described in **FIG.** 1. However, one or more steps involved in generating the reconstructions can be executed by any number of computing devices operating in the distributed computing system described in **FIG. 1****.** For instance, one or more computing devices can locally perform part or all of the operations to generate the reconstructions in **FIG. 9****.**

Volumes **902,** Displayed under "FDK reconstruction" are two views of a volume generated based on the stack of projections using an FDK process. The FDK process may be the same or similar to the FDK process described in **FIG. 4****.** As can be observed in both views of the FDK reconstruction, the voxel values of the area located outside of the SFoV may be undefined (e.g., are black). Additionally, an artifact (e.g., white line) may be present on the right side of both views of the volume.

Volumes **904,** displayed under "EFOV ART reconstruction," represent two views of a volume generated based on the stack of projections using an ART process. To generate the volumes **904,** the analytics server may make an initial estimation of the volume and then iteratively correct this estimation based on the stalk of projections during the ART process. As can be observed in both views of the ART reconstruction, an estimation of the portion of the volume that is outside the SFoV may be generated in an ART reconstruction. However, in some examples, this estimation may have decreased accuracy as distance from the SFoV increases. While the ART volume displays more of the patient than the FDK volume, the estimated values may be perceived as blurry (e.g., less accurate), particularly as the distance from the SFoV increases.

Volumes **906,** displayed under "Complete EFOV-DL ART reconstruction with single inference" are two views of a volume generated based on the stack of projections using extended field of view deep learning (EFoV-DL). The EFoV-DL process may be the same or similar to the process **600** of **FIG. 6****.** As can be observed in both views of the EFoV-DL volume, the EFoV-DL volume may display more clarity outside of the SFoV than the ART volume. For example, the EFOV-DL may include a clearer indication of where the patient stops and starts in both the top and bottom view. Furthermore, features such as the bones of the patient are more clearly defined in the EFOV-DL reconstruction than in the FDK reconstruction.

Volumes **908,** displayed under "Complete EFOV-DL ART reconstruction with recursive inference" are two reconstructions that are generated using extended field of view deep learning with one recursive inference. The EFoV-DL process may be similar to the process **600** of **FIG. 6****,** with a recursive inference at step **604.** In embodiments of EFoV-DL that include recursive inference, the model may generate the de-truncated volume as a corrected version of the truncated volume. The analytics server may then provide the detruncated volume to the model to cause the model to generate an updated de-truncated volume. In some examples, the updated de-truncated model may display a more accurate depiction of the patient. As can be observed in a comparison of the top view of the EFoV-DL volume and the EFoV-DL with recursive inference volume, the EFoV-DL with recursive inference volume may display more clarity, particularly in places that are far from the SFoV, such as the right and left edge of the patient. While the volume displayed in **FIG. 9** illustrates an example of EFoV-DL with one recursive inference, the recursive inference may be repeated multiple times.

### Reliability based on aleatoric and epistemic uncertainty

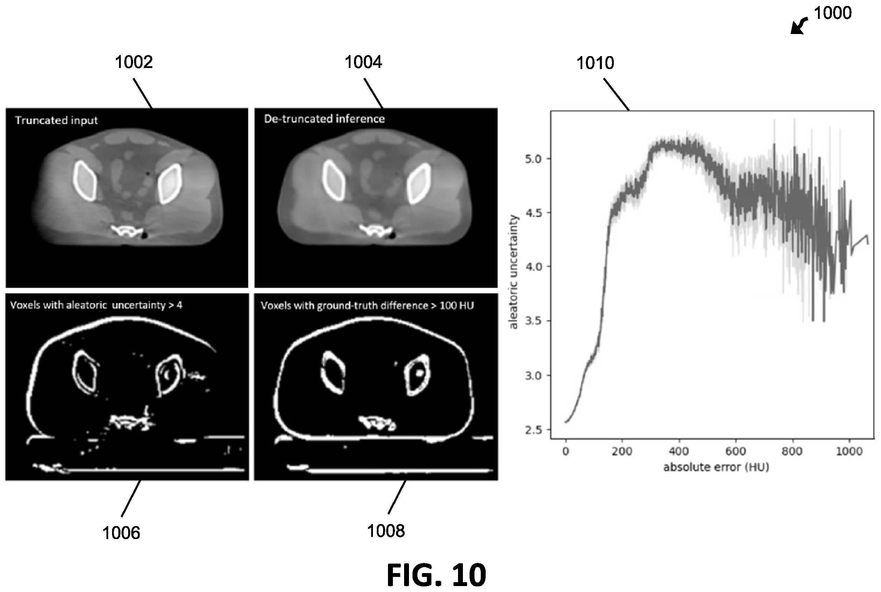

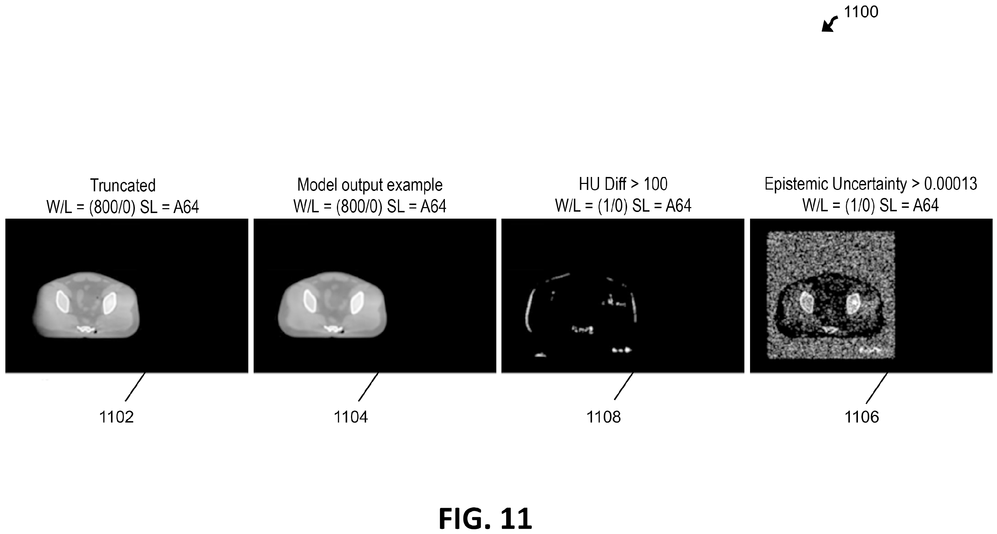

The model can have a level of confidence in its prediction (e.g., volumes **906).** The level of confidence may, for instance, indicate how reliable the output of the model is. Traditional methods of measuring confidence can measure a model prediction against a ground truth. For example, a non-truncated volume reconstructed from a stack of projections that has not been truncated can be compared to an associated de-truncated volume. However, in some examples, a ground truth (e.g., non-truncated volume) may not be available. In these examples, aleatoric and epistemic uncertainty can be used to indicate a level of confidence of the model's prediction. Aleatoric and epistemic uncertainty can measure uncertainty of data and model parameters, respectively, and can therefore be measured without a ground truth (e.g., non-truncated volume). As illustrated by **FIG. 10** and **FIG. 11****,** aleatoric and epistemic uncertainty can be correlated with large differences in model predictions and ground truth. As a result, aleatoric and epistemic uncertainty can be indicators of reliability of how reliable the output of the model is.

Aleatoric uncertainty may represent inherent uncertainty in data, and may originate from factors such as measurement inaccuracies, intrinsic variability within the system under observation, and limitations in the resolution or precision of data acquisition methods and may result in unavoidable ambiguity in the outcomes. Epistemic uncertainty may represent uncertainty arising from the model's lack of knowledge, and may arise from factors such as incomplete information, assumptions, or limitations in the model's structure. Both uncertainties may lead to potential inaccuracies in predictions or inferences. Analyzing the aleatoric and epistemic uncertainty of scans may indicate the reliability of predictions generated by a de-truncation model. In some examples, aleatoric and epistemic uncertainty can be used provide confidence maps that identify potential errors or hallucinations in the generated data, enhancing the clinical viability of the process **600** of **FIG. 6** for executing the trained neural network.

Referring to **FIG. 10****,** illustrated is a depiction **1000** of aleatoric uncertainty, according to an embodiment. The reconstructions include a truncated volume **1002** and a detruncated volume **1004.** The truncated volume **1002** may be a reconstruction of a truncated stack of projections captured by an imaging system (e.g., such as the patient **402** of **FIG. 4****)** where the patient was partially within the SFoV. The de-truncated volume **1004** may be generated based on the truncated stack of projections by a process that is the same or similar to the process **600** of **FIG. 6****.** The images also include an aleatoric uncertainty map **1006** and a de-truncated error representation **1008.** The aleatoric uncertainty map **1006** shows a visualization of the aleatoric uncertainty of the de-truncated mage. The de-truncated error representation **1008** shows a visualization of which voxels in the de-truncated inference differ largely from voxels in a full volume resulting from a scan where the patient is fully within the SFoV (e.g., a volume that may serve as a ground truth), as measured in HU.

Aleatoric uncertainty may reflect randomness in a prediction. In some examples, a higher aleatoric uncertainty may indicate that input data is more random (e.g., is noisier). As discussed above, a 3D reconstruction of a stack of projections may be represented by voxels. In described embodiments, aleatoric uncertainty may be generated for each voxel in a 3D construction. An aleatoric uncertainty score representing a level of inconsistency may be generated for each voxel value. In an example, the aleatoric uncertainty score may be a standard deviation of between equivalent voxel values in the plurality of 3D reconstructions.

As an example, a plurality of random augmentations (e.g., rotations, translations, flips across a 2D axis, scaling, and/or the like) of the truncated volume **1002** may be generated. For each random augmentation, a 3D reconstruction may be generated by a process that is the same or similar to the process **200** of **FIG. 2****.** In some examples, this may result in a detruncated volume that is the same or similar to the de-truncated volume **1004.** Reverse operations may then be applied to the resulting 3D reconstructions to transform them to the initial reference frame. As an example, a 3D reconstruction that was generated based on a version of the truncated input rotated 90° clockwise may be rotated 90° counterclockwise to be transformed to the initial reference frame. Based on the 3D reconstructions in the initial reference frame, a standard deviation of the voxel values between the plurality of 3D reconstructions may be generated and a natural logarithm may be applied to the value in each voxel to generate the aleatoric uncertainty score. In some examples, the aleatoric uncertainty map **1006** may be generated based on the aleatoric uncertainty scores of the voxels. In the aleatoric uncertainty map **1006,** a 2D representation of the aleatoric uncertainty scores for the voxels is displayed, where voxels with an aleatoric uncertainty score greater than 5 are represented by a white pixel while voxels with an aleatoric uncertainty score less than 5 are represented by a black pixel.

The de-truncated error representation **1008** displays a representation of the difference between a non-truncated volume (e.g., an ideal volume resulting from a scan where the patient was entirely within the SFoV) and the de-truncated volume **1004.** In some examples, the truncated volume may be generated based on the non-truncated volume. As an example, a non-truncated stack of projections may be truncated and reconstructed to generate the truncated volume. In this example, the non-truncated stack of projections can be a forward projection of an existing volume of a patient, where the patient was fully within the SFoV of the imaging system. The non-truncated volume can be generated based on the non-truncated stack of projections (e.g., forward projection of the existing volume) to reduce discrepancies resulting from possible simplifications during the forward projection process. The truncated volume **1002** may be generated based on the truncated stack of projections (e.g., trimmed version of the non-truncated stack of projections). In some embodiments, the de-truncated error representation **1008** may display a representation of the difference between the de-truncated volume **1004** and the non-truncated volume. By virtue of generating the de-truncated volume **1004** based on a modified version of the non-truncated stack of projections (e.g., as opposed to taking an additional scan with the patient repositioned), the system may reduce noise caused by small differences in variables such as position of the patient. In some examples, the detruncated error representation **1008** may represent the HU difference between voxels in the non-truncated volume and the de-truncated volume **1004.** As a result, the de-truncated error representation **1008** may represent the difference in indicated densities of the patient at each voxel. As illustrated in **FIG. 10****,** voxels with a difference of greater than 100 HU may be represented by white pixels while voxels with a difference of less than or equal to 100 HU may be represented by black pixels.

The uncertainty versus error graph **1010** may illustrate the correlation between aleatoric uncertainty and absolute error. As depicted in the uncertainty versus error graph **1010,** aleatoric uncertainty may have a positive correlation with absolute error (measure in HU), particularly for aleatoric uncertainty values less than a certain threshold (e.g., 4). As a result, voxels with higher aleatoric uncertainty (e.g., higher noise) may have higher error. This relationship can be observed in the similarities between the aleatoric uncertainty map **1006** and the de-truncated error representation **1008.** These two images may be observed to have at least some overlap, which indicates a correlation. The aleatoric uncertainty map **1006** can therefore indicate where voxel values predicted by the model may be unreliable (e.g., have a relatively high absolute error, as depicted in the de-truncated error representation **1008).** As discussed herein, ground truth volumes (e.g., non-truncated volumes, where the patient is fully within the SFoV) may not always be available due to the possible harmful effects of performing a scan (e.g., CT scans). Since aleatoric uncertainty **1006** can be determined without a ground truth volume, it can serve as an indicator of reliability in instances where there is no ground truth volume available.

Referring to **FIG. 11****,** illustrated is an example **1100** of an epistemic uncertainty graph. The truncated volume **1102** may be a volume reconstructed based on a truncated stack of projections and may be the same or similar to the truncated volume **1002** of **FIG. 10****.** The de-truncated volume **1104** may be generated as a correction of the truncated volume **1102** by a de-truncation model and may be the same or similar to the de-truncated volume **1004** of **FIG. 10****.** The de-truncated error representation **1108** may compare the de-truncated volume **1104** and a corresponding full volume (e.g., ground truth) to indicate which corresponding pairs of voxel values have a large difference in HU value and may be the same or similar to the detruncated error representation of **FIG. 10****.** The epistemic uncertainty map **1106** may display the epistemic uncertainty of voxel values in the de-truncated volume **1104.** As depicted in **FIG. 11****,** voxels with an epistemic uncertainty greater than .00013 may be represented by white pixels while voxels with an epistemic uncertainty less than .00013 may be represented by black pixels.

Epistemic uncertainty may reflect uncertainty within model parameters. In some examples, the uncertainty within model parameters may be simulated by selectively deactivating different subsets of neurons before inference. This process may be repeated M times with different de-activation subsets. In these examples, the variability in the model predictions may indicate uncertainty within model parameters. In the epistemic uncertainty map **1106,** epistemic uncertainty was analyzed using a dropout probability of .5 and an M value of 20. In this example, it was determined that 88.77% of voxels had an epistemic uncertainty below .00013, these voxels comprising a first group. It was further determined that 95.67% of the first group had a prediction error of less than 100 HU with respect to ground truth. As a result, it can be concluded that a large majority of the voxels in this executed example had a low level of epistemic uncertainty, which indicates that the de-truncation methods described herein may result in a volume with a high level of reliability.

In an embodiment, an analytics server, (e.g., that is the same or similar to the analytics server **114a** of **FIG. 1**) may generate confidence maps that identify potential errors or hallucinations in generated data. For example, in response to generating a de-truncated volume (e.g., by the process **600** of **FIG. 7****),** the analytics server may generate a confidence map displaying the epistemic and/or aleatoric uncertainty of the de-truncated volume. This map may be generated even in the absence of a ground truth volume. In some examples, the confidence map may identify potential errors and/or hallucinations. For example, the analytics server may identify potential error based on the epistemic and/or aleatoric uncertainty associated with a voxel in the de-truncated volume satisfying a threshold uncertainty. As a result, the analytics server may generate an indication of how reliable a de-truncation volume is, which can increase the clinical viability of described methods for de-truncation.

### Example workflows

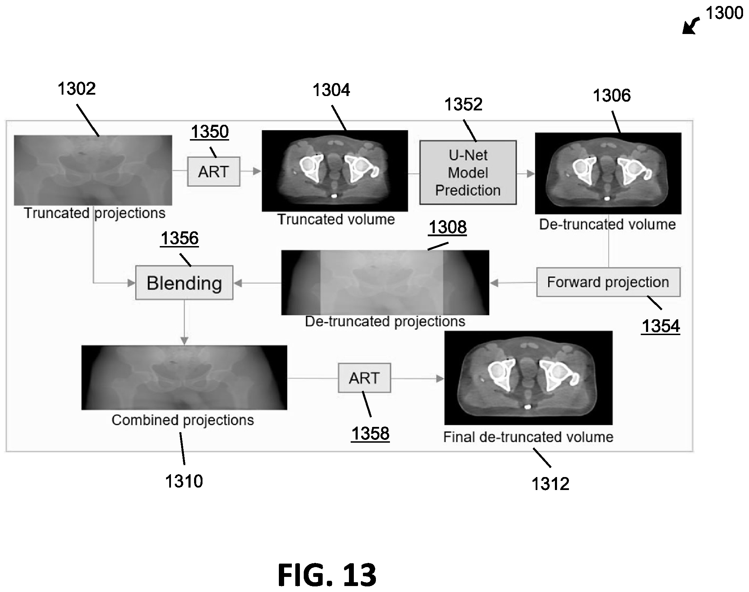

**FIGS. 12-14** represent non-limiting example workflows of systems and methods for training and executing a machine learning model to de-truncate volumes described herein. These workflows can describe example embodiments of training and executing a machine learning model to de-truncate volumes described herein. **FIG. 12** depicts a non-limiting example workflow of generating a training data set and training a machine learning model to generate de-truncated volumes, such as described in **FIG. 2****.** **FIG. 13** represent a non-limiting example workflow of executing the trained neural network to generate a de-truncated volume based on a truncated volume, such as described in **FIG. 6****.** **FIG. 14** represent a non-limiting example workflow of a process for de-truncation that includes scatter correction, such as described in **FIG. 6****.**

Referring to **FIG. 12****,** illustrated is a non-limiting example of the process **200** described in **FIG. 2****.** The workflow is described as being executed by an analytics server, which can be the same as, or similar to, the analytics server **114a** described in **FIG. 1****.** However, one or more steps of training workflow **1200** can be executed by any number of computing devices operating in the distributed computing system described in **FIG. 1****.** For instance, one or more computing devices can locally perform part or all of the operations described in **FIG. 12****.**

In the training workflow **1200,** a training data set may be generated. In some implementations, the training data set may comprise pairs of volumes, the pairs including a non-truncated (e.g., full volume) and a truncated volume that represent the same anatomy. This data set may be generated based on simulations of input volumes. As an example, the analytics server may generate a training data set based on simulations of pelvis volumes derived from CT scans. In some examples, it may be necessary to simulate training data sets due to a lack of existing pairs of truncated and full volumes. For example, many existing truncated scans may be the result of a patient being too large to be positioned such that they are fully in the SFoV of an imaging system. As a result, a full scan corresponding to such truncated scans may not exist. As another example, it may not be viable to perform additional scans of patient to obtain a pair to an existing full and/or truncated volume due to harmful effects of scans. For example, some imaging systems, such as a CT scanner, may capture stacks of projections based on delivering a radiation dose. While obtaining a scan for medical purposes may outweigh possible health risks of the radiation dose, it may not be viable to deliver an additional radiation dose to generate pairs for the purpose of model training. Systems and methods for generating a training data set, as described herein, may be used to simulate volume pairs for model training based on existing volumes.

The training workflow **1200** may start with an input volume **1202.** For example, the input volume **1202** may be reconstruction of a patient's internal structures based on a stack of projections obtained from an imaging system (e.g., such as the imaging system described in **FIG. 4****),** where the patient is fully within the SFoV of the imaging system. In some implementations, the analytics server may apply various geometric transformations (e.g. shifting, rotating, flipping, scaling, elastic deformation, and/or the like) to the input volume **1202.** In these implementations, the analytics server may generate a training data set pair including a truncated volume **1210** and a full volume **1206** based on each transformation of the input volume 1202. In some examples, the analytics server may apply various transformations (e.g., randomly, with a given probability, and or the like) to input volumes used to generate a training data set. Training a model may require significant a significant number of volume pairs. Otherwise, models, such as neural networks, may be overfit to the training dataset when provided an insufficient number of training pairs. As a result of applying transformations to initial volumes, the training workflow **1200** may greatly increase the number of volume pairs in a training data set. This may enhance the model's accuracy by offering more references in a wider range of positions.

At step **1250,** the analytics server may perform a forward projection on the input volume **1202.** The forward projection may simulate how the full volume would appear as a 2D projection on a detector (e.g., that is the same or similar to the detector **404** of **FIG. 4****).** As an example, the forward projection may simulate a cone-beam forward projection. In this example, the full volume may be projected onto a 2D plane such that the projection emulates what would be captured by a cone-shaped X-ray beam. In some implementations, a stack of simulated projections **1204** may be generated based on forward projections determined from various points around the full volume. In these implementations, the points may replicate the points that an imaging system would capture projections from. As a result, the stack of simulated projections **1204** may emulate a stack of projections produced by an imaging system. However, because the stack of simulated projections **1204** may be generated as a simplified mathematical simulation, it may not depict physical phenomena (e.g., noise, scatter, beam hardening, and/or the like). In some examples, this may result in the stack of simulated projections **1204** being less complex than a stack of projections produced by an imaging system. Using less complex data may, in some examples, make model training more efficient by reducing computational requirements and allowing the model to converge faster without being overwhelmed by details related to the physical phenomena. For example, the model may not be trained to correct voxel values based on collimation (e.g., geometric configuration of the signal from a transmitter within the imaging system) of imaging systems. As a result, the model may converge faster.

At step **1252,** the analytics server may trim the stack of simulated projections **1204** to generate a stack of truncated projections **1208.** In these implementations, the stack of simulated projections **1204** may be trimmed to emulate truncation. The stack of simulated projections **1204** may, for instance, be trimmed by a preset number of pixels on a side of the patient. As an example, 360 pixels may be trimmed off of the left side (e.g., patient's left) of each simulated projection in the stack of simulated projections **1204** to simulate the left side of the patient being partially outside of the SFoV. The stack of simulated projections **1204** may be truncated through various methods (e.g., at an angled line, irregular line, curved line, and/or the like). In some implementations, several truncated stacks of projections may be generated based on trimming the full stack of projections at different area. For example, a training data set may include truncated volumes associated with truncated stacks of simulated projections that have been trimmed in several different areas.

At step **1256,** the analytics server may generate a truncated volume **1210** based on the stack of truncated projections **1208.** For example, the analytics server may generate the truncated volume **1210** based on reconstructing (e.g., by ART, FDK, and/or the like) the stack of truncated projections **1208** into a 3D volume.

Similarly, at step **1254,** the analytics server may generate a full volume **1206** based on the stack of simulated projections **1204.** While the input volume **1202** may be a full 3D representation of the patient, generating the full volume **1206** based on the simulated stack of projections may ensure that the full volume **1206** is an appropriate ground truth for the truncated volume **1210.** As discussed above, the stack of simulated projections **1204** may be different than a stack of projections obtained from an imaging system. For example, simulated projections may be simplified projections that do not include details related to physical phenomena. This may impact aspects of volumes generated based on the stack of simulated projections **1204.** As a result, using the full volume **1206,** instead of the input volume **1202,** as a ground truth for the truncated volume **1210** may reduce unintended variance in a training pair, which can increase the quality of the training data set.

In some embodiments, the analytics may form a training pair including a full volume **1206** and a corresponding truncated volume **1210.** In some examples, the analytics server may generate a plurality of training pairs (e.g., generated from different full volumes, geometric transformations of full volumes, and/or the like). In some examples, the described process may be repeated with a large number of input volumes to generate a large training data set with many pairs of full and truncated volumes. In these examples, the input volumes may include input volumes generated based on more than one imaging technique. As an example, the training data set may include input volumes generate based on both CT scans and CBCT scans.

At step **1258,** the analytics server may train the model using the training data set comprising the full volume **1206** and the truncated volume **1210.** The model may be trained with a dataset including simulated pairs of full and truncated volumes to correct volumes that have been generated based on a truncated stack of projections. As an example, the model may be trained to generate a de-truncated volume based on a truncated volume, where the model corrects one or more voxel values of the truncated volume **1210,** such as the voxel values in the EFoV that have been estimated by ART using incomplete data. In some implementations, the training data set may be trimmed and/or normalized before training. In these implementations, the training data set may be trimmed to a first range of HU units (e.g., (-1000, 3000) HU units) and a second range of HU units (e.g., (-1000, 1000) HU units) may be normalized to values in a range of (0, 1).

In some embodiments, the model may be a convolutional neural network. The convolutional neural network may be a U-Net model that includes an encoder to capture high-level features and a decoder to recover spatial resolution. In some implementations, the model may be trained using adversarial training. Adversarial training may involve a generator (e.g., the model) and a discriminator. In some examples, the generator may incorporate attention gates into a standard U-Net model. Incorporating attention gates into the standard U-Net model may enhance sensitivity and prediction accuracy by allowing the generator to better focus on relevant image regions, which may may improve the quality of generated textures. In some examples, the discriminator may be a Generative Adversarial Network (GAN) that incorporates Attention-based Optimization Techniques (AOT). The discriminator may distinguish patches of real images from those generated by the model. As a result, the model may generate more convincing and realistic textures as it seeks to outmaneuver the discriminator. Furthermore, the model may generate more accurate volumes based on the discriminator detecting flaws such as artifacts (e.g., unwanted visual distortions) and/or hallucinations (e.g., false structures that may appear plausible). In some examples, HU matching may be performed between the truncated volume **1210** and the output of the generator model. This may allow the analytics server to identify discrepancies that could indicate errors such as hallucination.

In some embodiments, the analytics server may generate loss during the training process. In some examples, the analytics server may generate adversarial loss for the generator and/or discriminator. For example, the adversarial loss for the generator may represent how well the generator's output (e.g., the correction of the truncated volume **1210)** is able to deceive the discriminator into classifying the generated data as real. The adversarial loss for the discriminator may represent how well the discriminator can distinguish between real data (e.g., associated with full volume **1206)** and generated data (e.g., associated with truncated volume **1210).** Additionally or alternatively, the analytics server may generate accuracy loss based on the absolute differences between the generated pixel values and actual pixel values in the ground truth volumes. In addition to or in place of the adversarial loss and the accuracy loss, the analytics server may generate perceptual loss based on the difference (e.g., L1 distance) between the activation maps of the generated volumes and ground truth volumes. In some implementations, the model may be trained to minimize a combination (e.g. weighted or unweighted) of the adversarial loss for the generator, the perceptual loss, and/or the accuracy loss. Similarly, the discriminator may be trained to minimize adversarial loss for the discriminator (e.g., maximize adversarial loss for the generator) to improve classification accuracy of ground truth and generated volumes.

The losses may be expressed mathematically as functions of the pixels in the full volume and the de-truncated volume generated by the model. The model result z can be expressed as a function of *x,* a real image; m, a corresponding binary inpainting mask (e.g., 0 for known pixels and 1 for missing regions); ∘, pixel-wise multiplication; and G, the generator function, as shown below: $z = x ∘ \left(1-m\right) + G \left(x\right) ∘ m$

The model result z is therefore the sum of known pixels (x ∘ (1 - m)) and the output of the generator (*G*(*x*) ∘ m). Adversarial loss for the discriminator, ${L}_{D}^{adv}$*,* may be defined as the following function, where *σ* denotes the composition function of down-sampling and Gaussian filtering, applied to the complement of the inpainting mask, (1-m): ${L}_{D}^{adv} = {E}_{z ∼ {p}_{z}} \left[D\left(z\right)-σ{\left(1-m\right)}^{2}\right] + {E}_{z ∼ {p}_{data}} {\left[D\left(z\right)-1\right]}^{2}$

This adversarial loss for the discriminator may generate a soft patch-level mask for training. Adversarial loss for the generator, ${L}_{G}^{adv}$*,* can be defined as the following function: ${L}_{G}^{adv} = {E}_{z ∼ {p}_{z}} \left[{\left(D\left(z\right)-1\right)}^{2}∘m\right]$

L1 loss, L_{rec}, can be defined as the following function: ${L}_{rec} = {\left‖x-G\left(x\right)\right‖}_{1}$

Perceptual loss may be defined as the following function, where *ϕᵢ* refers to the activation map from the i-th layer of a pre-trained network and *Nᵢ* represents the number of elements in the activation map: ${L}_{per} = {\sum }_{i} \frac{{\left‖{ϕ}_{i}\left(x\right)-{ϕ}_{i}\left(z\right)\right‖}_{1}}{{N}_{i}}$

The overall optimization objective L may be summarized as the following combination of losses: $L = {λ}_{adv} {L}_{G}^{adv} + {λ}_{rec} {L}_{rec} + {λ}_{per} {L}_{per}$

As a result of balancing these three losses, the training workflow **1200** may achieve high-quality image generation. For example, de-truncated volumes may be generated based on the truncated volumes that are realistic and a close pixel-wise and feature match to the full volume **1206.**

Referring to **FIG. 13****,** depicted is non-limiting example of the process **600** of **FIG. 6****.** As an example, the workflow may utilize ART (e.g., iterative ART) reconstruction with active de-truncation based on deep learning. The workflow is described as being executed by an analytics server, which can be the same as, or similar to, the analytics server **114a** described in **FIG. 1****.** However, one or more steps of de-truncation workflow **1300** can be executed by any number of computing devices operating in the distributed computing system described in **FIG. 1****.** For instance, one or more computing devices can locally perform part or all of the operations described in **FIG. 13****.**

In the de-truncation workflow **1300,** the analytics server may receive a stack of truncated projections **1302.** The stack of truncated projections **1302** may be generated by an imaging system performing a scan of a patient that is not fully within the SFoV of the imaging system. As a result, the stack of truncated projections **1302** may not include the entire patient within every projection of in the stack of projections. While reconstructions methods such as ART or FDK may be utilized to reconstruct the portion of the patient within the SFoV, these reconstructions may have undefined values (e.g., for a reconstruction generated by FDK) or estimated values with limited accuracy (e.g., for ART).

At step **1350,** the stack of truncated projections **1302** may be reconstructed. As an example, the stack of truncated projections **1302** may be reconstructed using ART, since ART may maximize the use of information (e.g., including partial information about the parts of the patient outside of the SFoV) obtained from projections. As a result, a truncated volume **1304** may be generated. In some embodiments, values generated based on reconstructions of a 2D stack of projections may be a set of HU values representing the density of the patient at each voxel of the volume. The truncated volume **1304** may include estimated voxel values for portions of the patient that were outside of the SFoV of the imaging system in the stack of truncated projections **1302.** However, the accuracy of the voxel values may greatly decrease as distance from the border of the SFoV increases.

At step **1352,** a model (e.g., that is the same or similar to the model trained by the process **200** of **FIG. 2****)** may be utilized to correct the truncated volume **1304.** In these examples, the truncated volume **1304** may be provided to the model to cause the model to generate a de-truncated volume **1306.** The model may, for example, modify one or more voxel values of the truncated volume **1304** to generate a de-truncated volume **1306** that may be an improved (e.g., more accurate, more realistic, and/or the like) representation of the patient. In this example, after generating an initial de-truncated volume, the analytics server may perform additional HU-matching between the truncated volume **1304** and de-truncated volume **1306.** For example, the analytics server may perform HU-matching of the HU values for between voxels in the truncated volume **1304** and corresponding voxels in the de-truncated volume **1306.** Voxel values in the de-truncated volume **1306** may then be adjusted to better match voxel values in the truncated volume **1304** based on the HU-matching. In an example, the analytics server may select a region of interest. For example, the region of interest may be the portion of truncated volume **1304** and de-truncated volume **1306** that fall within the SFoV. In this example, the analytics may use a least squares approach to compute a linear transformation (e.g., with an offset and a slope) that may minimize the difference in HU values between truncated and de-truncated volumes. The linear transformation may, for instance, be applied to the de-truncated volume **1306** generated by the model.

At step **1354,** the analytics server may perform a forward projection on the detruncated volume **1306.** Step **1354** of the de-truncation workflow **1300** may be the same or similar to step **1250** of the training workflow **1200** of **FIG. 12****.** Similar to the forward projection process discussed in **FIG. 12****,** the forward projection process described herein may not simulate physical phenomena (e.g., noise, scatter, beam hardening, and/or the like. In some implementations, a stack of de-truncated projections **1308** may be generated based on forward projections determined from various points around the de-truncated volume **1306.** As an example, the analytics server may emulate projections under different angles (e.g., gantry angles representing rotational positions of an imaging system around a patient). In some examples, the analytics server may perform the forward projection such that the size of the virtual detector that is being simulated is larger (e.g., wider and/or taller) than the detector that captured the stack of truncated projections **1302.** In these examples, a certain number of columns and/or rows may be added to each projection. This may, for instance, provide more room in the projection to display the portion of the patient that is not within the SFoV.

At step **1356,** the stack of truncated projections **1302** may be blended with the stack of de-truncated projections **1308.** In these embodiments, the analytics may generate a stack of combined projections **1310** based on blending the stack of truncated projections **1302** with the stack of de-truncated projections **1308.** In some examples, the stack of truncated projections **1302** and/or the stack of de-truncated projections **1308** may be transformed from the transmission space to the attenuation space. For example, the projections may be physics-corrected and/or log-normalized before blending to transform them from the transmission space to the attenuation space. Additionally or alternatively, the stack of truncated projections **1302** and the stack of de-truncated projections **1308** may be transformed into sinograms before blending. In an example, the stack of combined projections **1310** may be a combined sinogram. For example, a truncated sinogram generated based on the stack of truncated projections **1302** and a de-truncated sinogram generated based on the stack of de-truncated projections **1308** may be blended into a combined sinogram. Sinograms may represent the patient in two-dimensions (e.g., from one viewpoint of the volume) based on the associated stack of projections. For example, the analytics server may combine multiple projections and organize them into a two-dimensional format that represents a particular viewpoint of the patient.

At step **1358,** the analytics server may generate a final de-truncated volume **1312** based on reconstructing the stack of combined projections **1310.** This final de-truncated volume **1312** may represent densities within the patient (e.g., internal structure of the patient) in three-dimensions by voxels comprising HU vales. As a result of the de-truncation workflow **1300,** the analytics server may generate a final de-truncated volume **1312** that is better (e.g., shows more detail, is more accurate, and/or the like) than the truncated volume **1304.**

Referring to **FIG. 14****,** depicted is non-limiting example of the process **600** of **FIG. 6** with scatter correction. As an example, a de-truncation workflow that is similar to the de-truncation workflow **1300** of **FIG. 13** may be performed where scatter correction (e.g., Acuros correction) may be performed to correct inaccuracies resulting from interactions between particles in an imaging signal (e.g., X-ray photons in a CT beam). The workflow is described as being executed by an analytics server, which can be the same as, or similar to, the analytics server **114a** described in **FIG.** 1. However, one or more steps of scatter-corrected de-truncation workflow **1400** can be executed by any number of computing devices operating in the distributed computing system described in **FIG. 1****.** For instance, one or more computing devices can locally perform part or all of the operations described in **FIG. 14****.**

In the scatter-corrected de-truncation workflow **1400,** the analytics server may receive a stack of truncated projections **1402.** At the step **1450,** a truncated volume **1404** may be generated by a process that is the same or similar to step **1350** of **FIG. 13****.** At step **1452,** a de-truncated volume **1406** may be generated by a process that is the same or similar to step **1352** of **FIG. 13****.**

In some examples, an imaging system (e.g., such as the imaging system of **FIG. 4****)** may transmit an imaging signal through a patient (e.g., such as the patient **402 of** **FIG. 4****)** to a detector (e.g., such as detector **404 of** **FIG. 4****)** to generate a stack of projections, based on which a volume representing the patient can be reconstructed. In some examples, the imaging signal may comprise particles (e.g., photons, protons, and/or the like) that can interact with the tissues of the patient (e.g., by absorption, scattering, and/or the like). While some interactions may provide useful information for generating the volume (e.g., Beer-Lambert Law interactions, photoelectric photon interactions, and/or the like), other interactions may result in projections that are difficult to reconstruct into an accurate volume. (e.g., Compton scatter, Rayleigh scatter, hardware-related quantum effects such as charge trapping, and/or the like). For example, interactions that impede reconstruction may cause projections that are difficult to reconstruct into volumes due to artifacts, noise, and/or inconsistencies between projections. In an example, some interactions of the imaging signal may result in inconsistent attenuation and scattering of the imaging signal through different tissues of the patient, which can produce artifacts (e.g., cupping) that negatively impact the accuracy of reconstructed volume. For example, the volume may provide a less accurate representation (e.g., in HU values) of the density the patient's internal structure and/or may display reduced contrast in some regions of the volume due to these artifacts. This may be a significant problem in cone-beam CT imaging, due to the cone-beam geometry of the imaging signal. In an embodiment, a scatter correction model (e.g., "Acuros") may be used to correct for scatter interactions. In this embodiment, the scatter correction model may correct a stack of projections based on accounting for different variables (e.g., tissue heterogeneity, scattering effects, collimated geometry, and/or the like). Additionally, or alternatively, other models may be executed to resolve other difficulties (e.g., hardware related quantum effects, polychromatic spectrum of sources with lack of elemental composition knowledge, and/or the like) resulting from interactions of particles.

At the step **1454,** the stack of truncated projections **1402** and the de-truncated volume **1406** may be provided to the scatter correction model to cause the scatter correction model to generate a corrected stack of projections **1410.** For example, the scatter correction model may correct the stack of truncated projections **1402** based on the de-truncated volume **1406,** thereby generating a corrected stack of projections **1410.** In some examples, the scatter correction model may correct the stack of truncated projections **1402** in the transmission (e.g., count) space. In these examples, physics corrections and/or log normalizations may be applied to the corrected stack of projections **1410** to transform the stack to the attenuation space.

At step **1456,** the analytics server may generate a stack of de-truncated projections **1408** based on the de-truncated volume **1406** by a process that is the same or similar to step **1354** of **FIG. 13****.**

At step **1458,** the analytics server may generate a combined stack of projections **1412** by a process that is the same or similar to step **1356** of **FIG. 13****.** For example, the combined stack of projections **1412** may be generated based on the corrected stack of projections **1410** and the stack of de-truncated projections **1408.** In an example, the corrected stack of projections **1410,** stack of de-truncated projections **1408,** and combined stack of projections **1412** may be in the attenuation space.

At step **1460,** the analytics server may generate a final de-truncated volume **1414** by a process that is the same or similar to step **1358** of **FIG. 13****.** In this example, the analytics server may utilize the de-truncated volume **1406** as an initial estimate and revise this initial estimate using ART. Additionally or alternatively, the analytics server may generate the final de-truncated volume **1414** by performing Penalized Likelihood (PL). PL is a statistical method that may be used in this application to penalize large differences between the volume estimation and a projection in the combined stack of projections **1412.** In an implementation, PL may utilize the combined stack of projections **1412** to perform one or more corrections on the de-truncated volume **1406.** In this example, the de-truncated volume **1406** may be generated using ART, and therefore have an estimate for voxel values that are associated with a part of the patient that is outside of the SFoV. As an alternative implementation, PL may generate a volume based only on the combined stack of projections **1412.** Before performing PL, the analytics server may transform the combined stack of projections **1412** into the transmission space. As an example, the following equation may be used to transform pixels from the attenuation space to the transmission space, where q is a pixel of the transmission space, r₀ is a measured air norm (e.g., representing a measurement of the radiation dose in the air that may be used to calibrate a CT imaging system), and p is a pixel in the attenuation space: $q = {r}_{0} {e}^{- p}$

In an example, the measured air norm r₀ may not be a constant. In this example, the measured air norm may differ depending on a projection and/or pixel of a projection within a stack. In examples where a combined projection in the combined stack of projections **1412** contains more columns and/or rows than the truncated projection, the air norm may be generated for pixels outside of the region corresponding to the truncated projection based on a computational model. For example, the computational model may determine the air norm within the EFoV region. In this example, the measured air norm may be used for pixels within the SFoV, and the generated air norm may be used for pixels within the EFoV. However, the computed air norm values may decrease due to a filter type associated with an imaging system. As an example, a CBCT system may utilize a beam-forming filter placed between the source and the patient to reduce unnecessary radiation. This may result in the air norm decreasing, in some cases to 0, in the region of a combined projection in the combined stack of projections **1412** corresponding to the EFoV. In this example, PL may not have an effect on pixels associated with a 0 air norm, since there are no values in the transmission space for these pixels. As a result, PL may only improve image quality in the SFoV. Alternatively, the analytics server may determine a constant air norm value for the EFoV region. For example, the value of the air norm on the edge of the SFoV may be extended through the EFoV. As a result, PL may be used to determine the final de-truncated volume **1414** based on the combined stack of projections **1412** without using the de-truncated volume as an initial estimate. It should be noted that PL may result in a visible ring of artifacts in the SFoV of the final de-truncated volume **1414.** This may be the result of insufficient penumbra compensation. This issue may be solved and/or improved by trimming the stack of truncated projections **1402** before starting the scatter-corrected de-truncation workflow **1400.**

### Example clauses

Further aspects of these teachings are provided by the subject matter of the following clauses (where it will be understood that any of these clauses can be combined with one or more of the other clauses as appropriate). Depending on the desired implementation, clause 2 may be combined with clause 1; clause 3 with clause 1 and/or clause 2; clause 4 with one or more of clauses 1-3; clause 5 with one or more of clauses 1-4; clause 6 with one or more of clauses 1-5, clause 7 with one or more of clauses 1-6; clause 8 with one or more of causes 1-7. This also applies to clause 9, which may be combined with one or more of clauses 10-15. This also applies to clause 16, which may be combined with one or more of clauses 17-20.

Clause 1. A method comprising: receiving, by at least one processor, a non-truncated volume comprising a three-dimensional representation of an anatomical region, the non-truncated volume representing imaging data within a full scan field of view (SFoV); simulating, by the at least one processor based on the non-truncated volume, an imaging system capturing a stack of projections comprising two-dimensional representations of the non-truncated volume, thereby generating a non-truncated stack of projections; simulating, by the at least one processor, truncation of the anatomical region by changing at least one visual attribute of the non-truncated stack of projections outside a predefined SFoV threshold, thereby generating a truncated stack of projections; reconstructing, by the at least one processor, a non-truncated training volume based on the non-truncated stack of projections and a truncated training volume based on the truncated stack of projections; forming, by the at least one processor, a set of training pairs comprising non-truncated training volumes paired with truncated training volumes, wherein the non-truncated volumes are designated as ground truth; and training, by the at least one processor, a machine learning model using the set of training pairs, wherein the machine learning model is configured to infer missing regions in truncated volumes.

Clause 2. The method of clause 1, wherein artificially simulating truncation comprises trimming a portion of a projection in the non-truncated stack of projections.

Clause 3. The method of clauses 1-2, wherein artificially simulating truncation emulates an imaging apparatus not fully capturing the anatomical region.

Clause 4. The method of clauses 1-3, wherein the machine learning model uses a composite loss function comprising at least one of voxel-wise reconstruction loss, perceptual loss, or adversarial loss.

Clause 5. The method of clauses 1-4, wherein the non-truncated volume is obtained using cone beam computed tomography (CBCT).

Clause 6. The method of clauses 1-5, wherein a plurality of training pairs within the set of training pairs are generated based on a plurality of geometric transformations of the non-truncated volume, further comprising: transforming, by the at least one processor, the non-truncated volume in response to receiving the non-truncated volume by applying a geometric transformation to the non-truncated volume.

Clause 7. The method of clauses 1-6, wherein training, by the at least one processor, comprises: generating, by the at least one processor, a de-truncated volume based on providing a truncated volume from the set of training pairs to the machine learning model to cause the machine learning model to generate the de-truncated volume; and evaluating, by the at least one processor, whether data representing a portion of the de-truncated volume is associated with measured data obtained from an imaging system or associated with the inferred missing region.

Clause 8. The method of clauses 1-7, further comprising: executing, by the at least one processor, Hounsfield Unit matching between the de-truncated volume and the truncated training volume.

Clause 9. A computer system comprising a computer-readable medium storage comprising a set of non-transitory instructions, that when executed, cause a processor to:
receive a non-truncated volume comprising a three-dimensional representation of an anatomical region, the non-truncated volume representing imaging data within a full scan field of view (SFoV); simulate an imaging system capturing a stack of projections comprising two-dimensional representations of the non-truncated volume, thereby generating a non-truncated stack of projections; simulate truncation of the anatomical region by changing at least one visual attribute of the non-truncated stack of projections outside a predefined SFoV threshold,
thereby generating a truncated stack of projections; reconstruct a non-truncated training volume based on the non-truncated stack of projections and a truncated training volume based on the truncated stack of projections; form a set of training pairs comprising non-truncated training volumes paired with truncated training volumes, wherein the non-truncated volume is designated as ground truth; and train a machine learning model using the set of training pairs,
wherein the machine learning model is configured to infer missing regions in truncated volumes.

Clause 10. The computer system of clause 9, wherein the set of non-transitory instructions further cause the processor to: generate a de-truncated volume based on providing a truncated volume from the set of training pairs to the machine learning model to cause the machine learning model to generate the de-truncated volume; and evaluate whether data representing a portion of the de-truncated volume is associated with measured data obtained from an imaging system or associated with the inferred missing region.

Clause 11. The computer system of clauses 9-10, wherein artificially simulating truncation comprises trimming a portion of the non-truncated stack of projections.

Clause 12. The computer system of clauses 9-11, wherein artificially simulating truncation emulates an imaging apparatus not fully capturing the anatomical region.

Clause 13. The computer system of clauses 9-12, wherein the machine learning model uses at least one of a composite loss function comprising voxel-wise reconstruction loss, perceptual loss, or adversarial loss.

Clause 14. The computer system of clause 9-13, wherein the non-truncated volume is obtained using cone beam computed tomography (CBCT).

Clause 15. The computer system of clauses 9-14, wherein a plurality of training pairs within the set of training pairs are generated based on a plurality of geometric transformations of the non-truncated volume, and wherein the instructions further cause the processor to: transform the non-truncated volume in response to receiving the non-truncated volume by applying a geometric transformation to the non-truncated volume.

Clause 16. The method of clauses 9-15, wherein the set of non-transitory instructions further cause the processor to execute Hounsfield Unit matching between the de-truncated volume and the truncated training volume.

Clause 17. A computer system comprising: a machine learning model; and a processor in communication with the machine learning model, the processor configured to:
receive a non-truncated volume comprising a three-dimensional representation of an anatomical region, the non-truncated volume representing imaging data within a full scan field of view (SFoV); simulate an imaging system capturing a stack of projections comprising two-dimensional representations of the non-truncated volume, thereby generating a non-truncated stack of projections; simulate truncation of the anatomical region by changing at least one visual attribute of the non-truncated stack of projections outside a predefined SFoV threshold,
thereby generating a truncated stack of projections; reconstruct a non-truncated training volume based on the non-truncated stack of projections and a truncated training volume based on the truncated stack of projections; form a set of training pairs comprising non-truncated training volumes paired with truncated training volumes, wherein the non-truncated volume is designated as ground truth; and train a machine learning model using the set of training pairs,
wherein the machine learning model is configured to infer missing regions in truncated volumes.

Clause 18. The computer system of clause 16, wherein the processor is further configured to: generate a de-truncated volume based on providing a truncated volume from the set of training pairs to the machine learning model to cause the machine learning model to generate the de-truncated volume; and evaluate whether data representing a portion of the de-truncated volume is associated with measured data obtained from the imaging system or associated with the inferred missing region.

Clause 19. The computer system of clause 16, wherein artificially simulating truncation comprises trimming a portion of the non-truncated stack of projections.

Clause 20. The computer system of clauses 16-17, wherein artificially simulating truncation emulates an imaging apparatus not fully capturing the anatomical region.

Clause 21. The computer system of clauses 16-18, wherein the machine learning model uses a composite loss function comprising at least one of voxel-wise reconstruction loss, perceptual loss, or adversarial loss.

Claims 22. The computer system of clause 16 that is configured to perform the method of any of clauses 1-8.

Clause 23: A computer program product comprising instructions which, when the program is executed by a computer, cause the computer to carry out the method of any of clauses 1-8.

Clause 24: A computer-readable medium comprising a set of instructions, that when executed by one or more processors, cause the one or more processor to carry out the method of any of clauses 1-8.

Further aspects of these teachings are provided by the subject matter of the following clauses (where it will be understood that any of these clauses can be combined with one or more of the other clauses as appropriate). Depending on the desired implementation, clause 2 may be combined with clause 1; clause 3 with clause 1 and/or clause 2; clause 4 with one or more of clauses 1-3; clause 5 with one or more of clauses 1-4; clause 6 with one or more of clauses 1-5, clause 7 with one or more of clauses 1-6. This also applies to clause 8, which may be combined with one or more of clauses 9-14. This also applies to clause 15, which may be combined with one or more of clauses 16-20.

Clause 1. A method comprising: receiving, by at least one processor, a truncated stack of projections comprising a set of two-dimensional representations of at least one anatomical structure of a patient, wherein the truncated stack of projections comprises missing or incomplete data corresponds to one or more regions outside a scan field of view; reconstructing, by the at least one processor, a truncated volume comprising a three-dimensional representation of the at least one anatomical structure based on the truncated stack of projections; executing, by the at least one processor, a trained neural network model using the truncated volume, the trained neural network model being configured to infer missing regions within the truncated volume and to predict a de-truncated volume; simulating, by the at least one processor, an imaging system capturing a stack of projections comprising two-dimensional representations of the de-truncated volume, thereby generating a de-truncated stack of projections; blending, by the at least one processor, the de-truncated stack of projections with the truncated stack of projections to form a combined stack of projections; and reconstructing, by the at least one processor, a combined volume from the combined stack of projections using an iterative reconstruction algorithm.

Clause 2. The method of clause 1, further comprising: simulating, by the at least one processor, at least one additional projection data beyond the scan field of view of the truncated volume.

Clause 3. The method of clauses 1-2, wherein the iterative reconstruction algorithm used for the combined volume is an algebraic reconstruction technique (ART).

Clause 4. The method of clauses 1-3, wherein the neural network model is trained on synthetic data pairs comprising truncated and non-truncated volumes.

Clause 5. The method of clauses 1-4, wherein the truncated stack of projections is obtained using cone beam computed tomography (CBCT) imaging.

Clause 6. The method of clauses 1-5, wherein blending the de-truncated stack of projections comprises determining a modification of the de-truncated stack of projections based on performing attenuation matching between the de-truncated stack of projections and the truncated stack of projections.

Clause 7. The method of clauses 1-6, further comprising: determining, by the at least one processor, a reliability of the de-truncated volume based on aleatoric or epistemic uncertainty.

Clause 8. A computer system comprising a computer-readable medium storage comprising a set of non-transitory instructions, that when executed, cause a processor to:
receive a truncated stack of projections comprising a set of two-dimensional representations of at least one anatomical structure of a patient, wherein the truncated stack of projections comprises missing or incomplete data corresponds to one or more regions outside a scan field of view; reconstruct a truncated volume comprising a three-dimensional representation of the at least one anatomical structure based on the truncated stack of proj ections; execute a trained neural network model using the truncated volume, the trained neural network model being configured to infer missing regions within the truncated volume and to predict a de-truncated volume; simulate an imaging system capturing a stack of projections comprising two-dimensional representations of the de-truncated volume, thereby generating a de-truncated stack of projections; blend the de-truncated stack of projections with the truncated stack of projections to form a combined stack of projections; and reconstruct a combined volume from the combined stack of projections using an iterative reconstruction algorithm.

Clause 9. The computer system of clause 8, wherein the set of non-transitory instructions further cause the processor to: simulate at least one additional projection data beyond the scan field of view of the truncated volume.

Clause 10. The computer system of clauses 8-9, wherein the iterative reconstruction algorithm used for the combined volume is an algebraic reconstruction technique (ART).

Clause 11. The computer system of clauses 8-10, wherein the neural network model is trained on synthetic data pairs comprising truncated and non-truncated volumes.

Clause 12. The computer system of clauses 8-11, wherein the truncated stack of projections is obtained using cone beam computed tomography (CBCT).

Clause 13. The computer system of clauses 8-12, wherein the et of non-transitory instructions that cause the processor to blend the de-truncated stack of projections with the truncated stack of projections further cause the processor to; determine a modification of the de-truncated stack of projections based on performing attenuation matching between the de-truncated stack of projections and the truncated stack of projections.

Clause 14. The computer system of clauses 8-13, wherein the set of non-transitory instructions further cause the processor to: determine a reliability of the de-truncated volume based on aleatoric or epistemic uncertainty.

Clause 15. A computer system comprising: a medical imaging system; and a processor in communication with the medical imaging system, the processor configured to: receive, from the medical imaging system, a truncated stack of projections comprising a set of two-dimensional representations of at least one anatomical structure of a patient, wherein the truncated stack of projections comprises missing or incomplete data corresponds to one or more regions outside a scan field of view; reconstruct a truncated volume comprising a three-dimensional representation of the at least one anatomical structure based on the truncated stack of projections; execute a trained neural network model using the truncated volume, the trained neural network model being configured to infer missing regions within the truncated volume and to predict a de-truncated volume; simulate an imaging system capturing a stack of projections comprising two-dimensional representations of the de-truncated volume, thereby generating a de-truncated stack of projections; blend the de-truncated stack of projections with the truncated stack of projections to form a combined stack of projections; and reconstruct a combined volume from the combined stack of projections using an iterative reconstruction algorithm.

Clause 16. The computer system of clause 15, the processor being further configured to: simulate at least one additional projection data beyond the scan field of view of the truncated volume.

Clause 17. The computer system of clauses 15-16, wherein the iterative reconstruction algorithm used for the combined volume is an algebraic reconstruction technique (ART).

Clause 18. The computer system of clauses 15-17, wherein the neural network model is trained on synthetic data pairs comprising truncated and non-truncated volumes.

Clause 19. The computer system of clauses 15-18, wherein the truncated volume is scatter corrected.

Clause 20. The computer system of clauses 15-19, the processor being further configured to: determine a reliability of the de-truncated volume based on aleatoric or epistemic uncertainty.

Claims 21. The computer system of clause 8 that is configured to perform the method of any of clauses 1-7.

Clause 22: A computer program product comprising instructions which, when the program is executed by a computer, cause the computer to carry out the method of any of clauses 1-7.

Clause 23: A computer-readable medium comprising a set of instructions, that when executed by one or more processors, cause the one or more processor to carry out the method of any of clauses 1-7.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the spirit or scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope and spirit being indicated by the following claims.

## Claims

1. A method comprising:
receiving, by at least one processor, a non-truncated volume comprising a three-dimensional representation of an anatomical region, the non-truncated volume representing imaging data within a full scan field of view, SFoV;
simulating, by the at least one processor based on the non-truncated volume, an imaging system capturing a stack of projections comprising two-dimensional representations of the non-truncated volume, thereby generating a non-truncated stack of projections;
simulating, by the at least one processor, truncation of the anatomical region by changing at least one visual attribute of the non-truncated stack of projections outside a predefined SFoV threshold, thereby generating a truncated stack of projections;
reconstructing, by the at least one processor, a non-truncated training volume based on the non-truncated stack of projections and a truncated training volume based on the truncated stack of projections;
forming, by the at least one processor, a set of training pairs comprising non-truncated training volumes paired with truncated training volumes, wherein the non-truncated volumes are designated as ground truth; and
training, by the at least one processor, a machine learning model using the set of training pairs, wherein the machine learning model is configured to infer missing regions in truncated volumes.

2. The method of claim 1, wherein artificially simulating truncation comprises trimming a portion of a projection in the non-truncated stack of projections.

3. The method of claim 1 or 2, wherein artificially simulating truncation emulates an imaging apparatus not fully capturing the anatomical region.

4. The method of claim 1, 2 or 3, wherein the machine learning model uses a composite loss function comprising at least one of voxel-wise reconstruction loss, perceptual loss, or adversarial loss.

5. The method of any one of claims 1 to 4, wherein the non-truncated volume is obtained using cone beam computed tomography, CBCT.

6. The method of any one of claims 1 to 5, wherein a plurality of training pairs within the set of training pairs are generated based on a plurality of geometric transformations of the non-truncated volume, further comprising:
transforming, by the at least one processor, the non-truncated volume in response to receiving the non-truncated volume by applying a geometric transformation to the non-truncated volume.

7. The method of any one of claims 1 to 6, wherein training, by the at least one processor, comprises:
generating, by the at least one processor, a de-truncated volume based on providing a truncated volume from the set of training pairs to the machine learning model to cause the machine learning model to generate the de-truncated volume; and
evaluating, by the at least one processor, whether data representing a portion of the de-truncated volume is associated with measured data obtained from an imaging system or associated with the inferred missing region.

8. The method of claim 7, further comprising:
executing, by the at least one processor, Hounsfield Unit matching between the de-truncated volume and the truncated training volume.

9. A computer-readable storage medium comprising a set of non-transitory instructions, that when executed, cause a processor to:
receive a non-truncated volume comprising a three-dimensional representation of an anatomical region, the non-truncated volume representing imaging data within a full scan field of view, SFoV;
simulate an imaging system capturing a stack of projections comprising two-dimensional representations of the non-truncated volume, thereby generating a non-truncated stack of projections;
simulate truncation of the anatomical region by changing at least one visual attribute of the non-truncated stack of projections outside a predefined SFoV threshold, thereby generating a truncated stack of projections;
reconstruct a non-truncated training volume based on the non-truncated stack of projections and a truncated training volume based on the truncated stack of projections;
form a set of training pairs comprising non-truncated training volumes paired with truncated training volumes, wherein the non-truncated volume is designated as ground truth; and
train a machine learning model using the set of training pairs, wherein the machine learning model is configured to infer missing regions in truncated volumes.

10. The computer-readable storage medium of claim 9, wherein the set of non-transitory instructions further cause the processor to:
generate a de-truncated volume based on providing a truncated volume from the set of training pairs to the machine learning model to cause the machine learning model to generate the de-truncated volume; and
evaluate whether data representing a portion of the de-truncated volume is associated with measured data obtained from an imaging system or associated with the inferred missing region.

11. The computer-readable storage medium of claim 9 or 10, wherein artificially simulating truncation comprises trimming a portion of the non-truncated stack of projections.

12. The computer-readable storage medium of claim 9, 10 or 11, wherein artificially simulating truncation emulates an imaging apparatus not fully capturing the anatomical region.

13. The computer-readable storage medium of claim 9, 10, 11 or 12, wherein the set of non-transitory instructions further cause the processor to perform the method of any one of claims 4, 5 or 6, or claim 8 when dependent on claim 10.

14. A computer system comprising:
a machine learning model; and
a processor in communication with the machine learning model, the processor configured to:
receive a non-truncated volume comprising a three-dimensional representation of an anatomical region, the non-truncated volume representing imaging data within a full scan field of view, SFoV;
simulate an imaging system capturing a stack of projections comprising two-dimensional representations of the non-truncated volume, thereby generating a non-truncated stack of projections;
simulate truncation of the anatomical region by changing at least one visual attribute of the non-truncated stack of projections outside a predefined SFoV threshold, thereby generating a truncated stack of projections;
reconstruct a non-truncated training volume based on the non-truncated stack of projections and a truncated training volume based on the truncated stack of projections;
form a set of training pairs comprising non-truncated training volumes paired with truncated training volumes, wherein the non-truncated volume is designated as ground truth; and
train a machine learning model using the set of training pairs, wherein the machine learning model is configured to infer missing regions in truncated volumes.

15. The computer system of claim 14, configured to perform the method of any one of claims 2 to 8 .
